(19) Europäisches Patentamt
European Patent Office
Office européen des brevets

(11) Veröffentlichungsnummer: **0 071 150**
**B1**

(12) **EUROPÄISCHE PATENTSCHRIFT**

(45) Veröffentlichungstag der Patentschrift: **03.07.85**

(21) Anmeldenummer: **82106501.8**

(22) Anmeldetag: **19.07.82**

(51) Int. Cl.⁴: **C 07 D 215/22**, C 07 D 209/34, A 61 K 31/47, A 61 K 31/40, C 07 D 401/02, C 07 D 409/02, A 61 K 31/455

(54) **Sulfimine, deren Salze und deren Herstellung sowie diese Verbindungen enthaltende Arzneimittel.**

(30) Priorität: **25.07.81 DE 3129444**
**29.10.81 DE 3142904**

(43) Veröffentlichungstag der Anmeldung: **09.02.83 Patentblatt 83/6**

(45) Bekanntmachung des Hinweises auf die Patenterteilung: **03.07.85 Patentblatt 85/27**

(84) Benannte Vertragsstaaten: **AT BE CH DE FR IT LI LU NL SE**

(56) Entgegenhaltungen:
**EP - A - 0 003 771**
**EP - A - 0 006 506**
**DE - A - 2 502 156**
**DE - A - 2 527 937**

**Die Akte enthält technische Angaben, die nach dem Eingang der Anmeldung eingereicht wurden und die nicht in dieser Patentschrift enthalten sind.**

(73) Patentinhaber: **Dr. Karl Thomae GmbH, Postfach 1755, D-7950 Biberach (Riss) (DE)**

(72) Erfinder: **Müller, Erich, Dr., Talfeldstrasse 34, D-7950 Biberach 1 (DE)**
Erfinder: **Nickl, Josef, Dr. Dipl.-Chem., Silcherstrasse 8, D-7950 Biberach 1 (DE)**
Erfinder: **Narr, Berthold, Dr. Dipl.-Chem., Obere Au 5, D-7950 Biberach 1 (DE)**
Erfinder: **Roch, Josef, Dr. Dipl.-Chem., Stecherweg 19, D-7950 Biberach 1 (DE)**
Erfinder: **Haarmann, Walter, Dr., Schlierholzweg 27, D-7950 Biberach 1 (DE)**
Erfinder: **Weisenberger, Johannes M., Dr. Dipl.-Chem., Haydnweg 5, D-7950 Biberach 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 071 150 B1

ACTORUM AG

## Beschreibung

In der EP-A-0.003.771 werden bereits Carbostyril- und Oxindolderivate beschrieben, welche neben einer positiv inotropen Wirkung insbesondere antithrombotische Wirkungen aufweisen.

Es wurden nun Sulfimine der allgemeinen Formel:

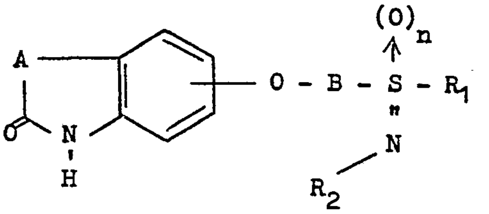

(I)

welche sich von den Verbindungen der EP-A-0.003.771 durch den $R_2-N=$-Substituenten an der Schwefelfunktion unterscheiden, gefunden.

Gegenstand der vorliegenden Erfindung sind die neuen Sulfimine der obigen allgemeinen Formel I, deren Salze mit starken Säuren, insbesondere deren physiologisch verträgliche Säureadditionssalze, diese Verbindungen enthaltende Arzneimittel und Verfahren zu ihrer Herstellung.

In der obigen allgemeinen Formel I bedeutet

n die Zahl 0 oder 1,

A eine gegebenenfalls durch eine oder zwei Alkylgruppen mit jeweils 1 bis 3 Kohlenstoffatomen substituierte Methylen-, Vinylen- oder Äthylengruppe,

B eine geradkettige oder verzweigte Alkylengruppe mit 2 bis 6 Kohlenstoffatomen,

$R_1$ eine gegebenenfalls durch eine Phenylgruppe substituierte Alkylgruppe mit 1 bis 3 Kohlenstoffatomen oder eine Phenylgruppe, wobei der Phenylkern jeweils durch eine Alkylgruppe mit 1 bis 4 Kohlenstoffatomen, ein Halogenatom, eine Alkoxygruppe mit 1 bis 3 Kohlenstoffatomen, eine Cyclohexyl-, Phenyl- oder Halogenphenylgruppe substituiert sein kann, eine Alkylgruppe mit 4 bis 7 Kohlenstoffatomen, eine Cycloalkylgruppe mit 3 bis 7 Kohlenstoffatomen, eine durch Alkylgruppen mit 1 bis 4 Kohlenstoffatomen, Alkoxygruppen mit 1 bis 3 Kohlenstoffatomen und/oder Halogenatome di- oder trisubstituierte Phenyl- oder disubstituierte Hydroxy- oder Aminophenylgruppe, wobei die Substituenten des Phenylkerns gleich oder verschieden sein können, eine gegebenenfalls durch eine Alkoxygruppe mit 1 bis 3 Kohlenstoffatomen substituierte Naphthylgruppe oder eine Pyridylgruppe und

$R_2$ ein Wasserstoffatom, eine gegebenenfalls durch eine Methoxygruppe substituierte Alkanoylgruppe mit 1 bis 8 Kohlenstoffatomen, eine gegebenenfalls durch ein Halogenatom, eine Cyangruppe oder eine Alkylgruppe mit 1 bis 4 Kohlenstoffatomen substituierte Benzoyl- oder Phenylsulfonylgruppe, eine Alkoxycarbonylgruppe mit insgesamt 2 bis 4 Kohlenstoffatomen, eine gegebenenfalls durch eine Chlorphenylgruppe, durch eine oder zwei Methylgruppen substituierte Aminocarbonylgruppe, eine Naphthoyl-, Pinanoyl-, Camphersulfonyl-, Pentamethyl-phenylsulfonyl-, Pyridinoyl- oder Thenoylgruppe.

Unter den bei der Definition des Restes $R_1$ erwähnten Halogenatomen ist insbesondere ein Fluor-, Chlor- oder Bromatom zu verstehen.

Für die bei der Definition der Reste A, B, $R_1$ und $R_2$ eingangs erwähnten Bedeutungen kommt beispielsweise

für A die Bedeutung der Methylen-, Methylmethylen-, Dimethylmethylen-, Diäthylmethylen-, Dipropylmethylen, Vinylen-, Methyl-vinylen- oder Äthylengruppe,

für B die der Äthylen, n-Propylen-, n-Butylen-, n-Pentylen-, n-Hexylen-, 1-Methyl-äthylen-, 2-Methyl-äthylen-, 1-Methyl-n-propylen-, 2-Methyl-n-propylen-, 3-Methyl-n-propylen-, 1-Methyl-n-butylen-, 2-Methyl-n-butylen-, 3-Methyl-n-butylen-, 4-Methyl-n-butylen, 1-Methyl-n-pentylen-, 2-Methyl-n-pentylen-, 3-Methyl-n-pentylen-, 4-Methyl-n-pentylen-, 5-Methyl-n-pentylen-, 1,1-Dimethyl-äthylen-, 1,2-Dimethyl-äthylen-, 2,2-Dimethyl-äthylen-, 1,1-Dimethyl-n-propylen-, 2,2-Dimethyl-n-propylen-, 3,3-Dimethyl-n-propylen-, 1,2-Dimethyl-n-propylen-, 1,3-Dimethyl-n-propylen-, 1,1-Dimethyl-n-butylen-, 2,2-Dimethyl-n-butylen-, 3,3-Dimethyl-n-butylen-, 4,4-Dimethyl-n-butylen-, 1,2-Dimethyl-n-butylen-, 1,3-Dimethyl-n-butylen-, 1,4-Dimethyl-n-butylen-, 2,3-Dimethyl-n-butylen-, 1-Äthyl-äthylen-, 2-Äthyl-äthylen-, 1-Äthyl-n-propylen-, 2-Äthyl-n-propylen-, 3-Äthyl-n-propylen-, 1-Äthyl-n-butylen-, 2-Äthyl-n-butylen-, 3-Äthyl-n-butylen-, 4-Äthyl-n-butylen-, 1-Methyl-2-äthyl-äthylen-, 1-Methyl-2-äthyl-n-propylen-, 1-Methyl-3-äthyl-n-propylen-, 1-Methyl-2-propyl-äthylen-, 1-Propyl-äthylen-, 1-Butyl-äthylen- oder 1-Propyl-n-propylengruppe,

für $R_1$ die der Methyl-, Äthyl-, Propyl-, Isopropyl-, Butyl-, Isobutyl-, tert.-Butyl-, Pentyl-, Neopentyl-, tert.-Pentyl-, Hexyl-, Heptyl-, Benzyl-, 1-Phenyläthyl-, 2-Phenyläthyl-, 1-Phenylpropyl-, 3-Phenylpropyl-, Fluorbenzyl-, Chlorbenzyl-, Brombenzyl-, Methylbenzyl-, Isopropylbenzyl-, Methoxybenzyl-, Äthoxybenzyl-, Cyclopropyl-, Cyclobutyl-, Cyclopentyl-, Cyclohexyl-, Cycloheptyl-, Phenyl-, Fluorphenyl-, Chlorphenyl-, Bromphenyl-, Methylphenyl-, Dimethylphenyl-, Isopropylphenyl-, tert.-Butylphenyl-, Methoxyphenyl-, Äthoxyphenyl-, Propoxyphenyl-, Cyclohexylphenyl-, Biphenylyl-, Fluorphenyl-phenyl-, Chlorphenyl-phenyl-, Difluorphenyl-, Dichlorphenyl-, Dibromphenyl-, Dimethoxyphenyl-, Methoxychlorphenyl-, Methoxy-bromphenyl-, Methyl-tert.-butyl-phenyl-, Methyl-chlorphenyl-, Methyl-bromphenyl-, tert.-Butyl-bromphenyl-, Dichloraminophenyl-, Dibrom-aminophenyl-, Dimethyl-aminophenyl-, Dichlor-hydroxyphenyl-, Dibrom-hydroxyphenyl-, Dimethyl-hydroxyphenyl-, Di-tert.-butyl-hydroxyphenyl-, Trimethoxyphenyl-, Naphthyl-, Methoxynaphthyl- oder Pyridylgruppe und

für $R_2$ die des Wasserstoffatoms, der Formyl-, Acetyl-, Propionyl-, Pivaloyl-, Pentanoyl-, Hexanoyl-, Heptanoyl-, Octanoyl, Nonanoyl-, Methoxyacetyl-, Methoxypropionyl-, Pinanoyl-, Benzoyl-, Fluorbenzoyl-, Chlorbenzoyl-, Brombenzoyl-, Cyanobenzoyl-, Methylbenzoyl-, Äthylbenzoyl-, Isopropylbenzoyl-, tert.-Butylbenzoyl-, Difluorbenzoyl-, Dichlorbenzoyl-, Dimethylbenzoyl-, Trimethylbenzoyl-, Naphthoyl-, Pyridinoyl-, Thenoyl-, Acetoxy-benzoyl-, Hydroxysulfonyl-, Methylsulfonyl-, Äthylsulfonyl-, Camphersulfonyl-, Phenylsulfonyl-, Methylphenyl-sulfonyl-, Fluorphenylsulfonyl-, Chlorphenylsulfonyl-, Bromphenylsulfonyl-, Pentamethylphenylsulfonyl-, Naphthylsulfonyl-, Methoxycarbonyl-, Äthoxycarbonyl-, Propoxycarbonyl-, Isopropoxycarbonyl-, Benzyloxycarbonyl-, Aminocarbonyl-, Methylaminocarbonyl-, Dimethylaminocarbonyl-, Phenylaminocarbonyl- oder Chlorphenylaminocarbonylgruppe in Betracht.

Bevorzugte Verbindungen der obigen allgemeinen Formel I sind diejenigen, in denen

$R_2$ wie vorstehend erwähnt definiert ist,

n die Zahl 0 oder 1,

A eine Dimethylmethylen-, Vinylen- oder Äthylengruppe,

B eine geradkettige Alkylengruppe mit 3 bis 5 Kohlenstoffatomen und

$R_1$ eine Alkylgruppe mit 1 bis 6 Kohlenstoffatomen, eine Benzyl-, Phenyläthyl-, Cyclohexyl-, Naphthyl-, Methoxy-naphthyl- oder Pyridylgruppe, eine gegebenenfalls durch eine Alkylgruppe mit 1 bis 4 Kohlenstoffatomen, eine Methoxy-, Cyclohexyl-, Phenyl- oder Fluorphenylgruppe, ein Fluor-, Chlor- oder Bromatom substituierte Phenylgruppe, eine durch Alkylgruppen mit 1 bis 4 Kohlenstoffatomen, Methoxygruppen, Chlor- und/oder Bromatome disubstituierte Phenylgruppe, wobei die Substituenten des Phenylkerns gleich oder verschieden sein können, oder eine durch zwei Chlor- oder Bromatome, zwei Methoxygruppen oder zwei Alkylgruppen mit jeweils 1 bis 4 Kohlenstoffatomen substituierte Aminophenyl-, Hydroxyphenyl- oder Methoxyphenylgruppe bedeuten, und deren Salze mit starken Säuren.

Besonders bevorzugte Verbindungen der obigen allgemeinen Formel I sind jedoch diejenigen, in denen

n die Zahl 1,

A eine Dimethylmethylen-, Vinylen- oder Äthylengruppe,

B eine n-Butylengruppe,

$R_1$ eine Methyl- oder Methoxynaphthylgruppe, eine gegebenenfalls durch eine Methoxygruppe, ein Fluor- oder Chloratom substituierte Phenylgruppe, eine durch zwei Chlor- oder Bromatome substituierte Phenylgruppe, eine Methylbromphenyl-, 4-Amino-3,5-dibrom-phenyl- oder Di-tert.-butyl-hydroxy-phenylgruppe und

$R_2$ ein Wasserstoffatom, eine Alkanoylgruppe mit 1 bis 3 Kohlenstoffatomen oder eine gegebenenfalls durch eine Alkylgruppe mit 1 bis 4 Kohlenstoffatomen substituierte Benzoyl- oder Phenylsulfonylgruppe bedeuten, und deren physiologisch verträgliche Salze mit starken Säuren.

Erfindungsgemäss erhält man die neuen Verbindungen nach folgenden Verfahren:

a) Zur Herstellung einer Verbindung der allgemeinen Formel I, in der n die Zahl 1 und $R_2$ ein Wasserstoffatom darstellen:

Umsetzung eines Sulfoxids der allgemeinen Formel:

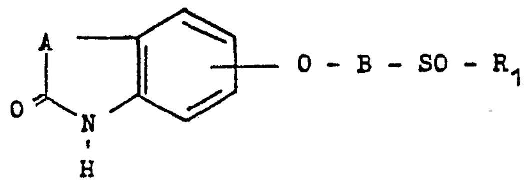
(II)

in der

A, B und $R_1$ wie eingangs definiert sind, mit gegebenenfalls im Reaktionsgemisch gebildeter Stickstoffwasserstoffsäure.

Die Umsetzung wird zweckmässigerweise in einem Lösungsmittel oder Lösungsmittelgemisch wie Methylenchlorid, Dimethylformamid oder Tetrahydrofuran bei Temperatur zwischen 0 und 40° C, vorzugsweise bei Temperaturen zwischen 10 und 35° C, durchgeführt. Besonders vorteilhaft wird die Umsetzung mit einem Alkaliazid, z.B. Natriumazid, und Polyphosphorsäure als Lösungsmittel durchgeführt.

b) Zur Herstellung einer Verbindung der allgemeinen Formel I, in der $R_2$ ein Wasserstoffatom darstellt:

Umsetzung eines Sulfoxids der allgemeinen Formel:

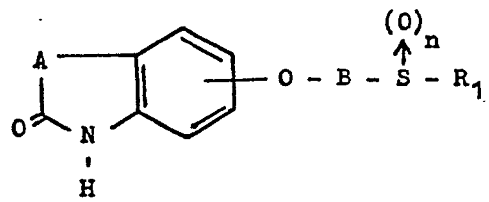
(IIa)

in der

A, B, n und $R_1$ wie eingangs definiert sind, mit einer Verbindung der allgemeinen Formel:

$$H_2N-O-X-R_3 \qquad (III)$$

in der

X eine Carbonyl- oder Sulfonylgruppe und

$R_3$ eine in o-Stellung disubstituierte Arylgruppe wie eine 2,4,6-Trimethylphenyl- oder 2,4,6-Triisopropylphenylgruppe oder auch eine Hydroxygruppe darstellt, wenn X die Sulfonylgruppe bedeutet.

Die Umsetzung wird zweckmässigerweise in einem Lösungsmittel oder Lösungsmittelgemisch wie Methylenchlorid, Chloroform, Dimethylformamid, Tetrahydrofuran oder Dioxan bei Temperaturen zwischen 0 und 50° C, vorzugsweise jedoch bei Temperaturen zwischen 5 und 40° C, durchgeführt. Besonders vorteilhaft wird die Umsetzung jedoch in der Weise durchgeführt, dass eine Verbindung der allgemeinen Formel III ohne ihre vorherige Isolierung eingesetzt wird bzw. im Reaktionsgemisch hergestellt wird.

c) Zur Herstellung einer Verbindung der allgemeinen Formel I, in der n die Zahl 1 darstellt:

Oxidation einer Mercaptoverbindung der allgemeinen Formel:

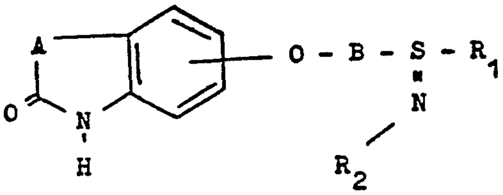 (IV)

in der

A, B, $R_1$ und $R_2$ wie eingangs definiert sind.

Die Oxidation wird vorzugsweise in einem Lösungsmittel oder Lösungsmittelgemisch, z.B. in Wasser, Wasser/Pyridin, Methanol, Äthanol, Aceton, Ameisensäure, Eisessig, Trifluoressigsäure oder verdünnter Schwefelsäure, je nach dem verwendeten Oxidationsmittel bei Temperaturen zwischen −80 und 100° C durchgeführt. Besonders vorteilhaft wird die Umsetzung mit einem Äquivalent des verwendeten Oxidationsmittels durchgeführt, z.B. mit Wasserstoffperoxid in Eisessig oder Ameisensäure bei 0 bis 20° C oder in Aceton bei 0 bis 60° C, mit einer Persäure wie Perameisensäure in Eisessig oder Trifluoressigsäure bei 0 bis 50° C oder mit Natriummetaperjodat in wässerigem Methanol oder Äthanol bei 15 bis 25° C.

d) Zur Herstellung einer Verbindung der allgemeinen Formel I, in der $R_2$ kein Wasserstoffatom darstellt:

Acylierung einer Verbindung der allgemeinen Formel:

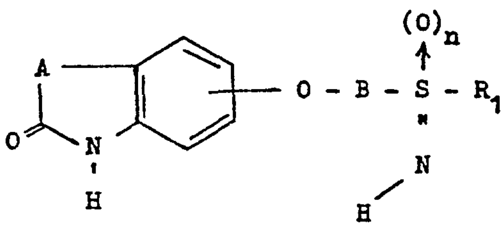 (V)

in der

n, A, B und $R_1$ wie eingangs definiert sind.

Die Umsetzung wird zweckmässigerweise in einem Lösungsmittel oder Lösungsmittelgemisch wie Wasser, Methylenchlorid, Chloroform, Äther, Tetrahydrofuran, Dioxan oder Dimethylformamid mit einem entsprechenden Acylierungsmittel, z.B. mit einer Säure in Gegenwart eines die Säure aktivierenden oder wasserentziehenden Mittels wie Thionylchlorid, mit deren Anhydriden wie Essigsäureanhydrid, mit deren Estern wie p-Toluolsulfonsäureäthylester oder Kohlensäurediäthylester, mit deren Halogeniden wie Acetylchlorid, Chlorameisensäureäthylester oder p-Toluolsulfonsäurechlorid oder mit einem entsprechenden Isocyanat, wobei diese gegebenenfalls auch als Lösungsmittel dienen können, gegebenenfalls in Gegenwart einer anorganischen oder tertiären organischen Base, wie Natriumhydroxid, Kaliumcarbonat, Triäthylamin oder Pyridin, wobei die letzteren gleichzeitig auch als Lösungsmittel dienen können, bei Temperaturen zwischen −25 und 100° C, vorzugsweise jedoch bei Temperaturen zwischen −10 und 80° C, durchgeführt.

e) Umsetzung einer Hydroxyverbindung der allgemeinen Formel:

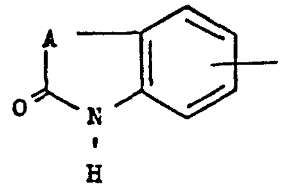 (VI)

in der

A wie eingangs definiert ist, oder deren Salze mit anorganischen oder tertiären organischen Basen mit einer Verbindung der allgemeinen Formel:

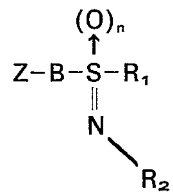 (VII)

in der

n, $R_1$, $R_2$ und B wie eingangs definiert sind und

Z eine nukleophil austauschbare Gruppe wie ein Halogenatom oder einen Sulfonsäureesterrest, z.B. ein Chlor-, Brom-, Jodatom, eine p-Toluolsulfonyloxy- oder Methansulfonyloxygruppe, darstellt.

Die Umsetzung wird zweckmässigerweise in einem geeigneten Lösungsmittel oder Lösungsmittelgemisch wie Dioxan, Tetrahydrofuran, Chloroform oder Toluol, vorzugsweise jedoch in einem wasserfreien aprotischen Lösungsmittel wie Aceton, Dimethylformamid oder Dimethylsulfoxid, gegebenenfalls in Gegenwart einer Alkalibase wie Natriumkarbonat, Kaliumkarbonat oder Natriumhydroxid bei Temperaturen zwischen 0° C und der Siedetemperatur des verwendeten Lösungsmittels, z.B. bei Temperaturen zwischen 0 und 100° C, vorzugsweise jedoch bei Temperaturen zwischen 10 und 50° C, durchgeführt. Die Umsetzung kann jedoch auch ohne Lösungsmittel durchgeführt werden.

f) Zur Herstellung einer Verbindung der allgemeinen Formel I, in der n die Zahl 0 darstellt:

Umsetzung eines Thioäthers der allgemeinen Formel:

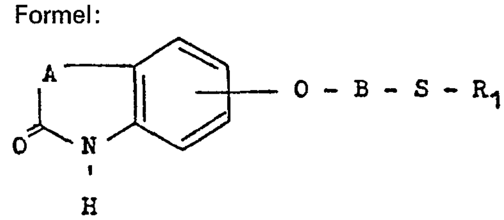 (VIII)

in der

A, B und $R_1$ wie eingangs definiert sind, mit einem Amid der allgemeinen Formel:

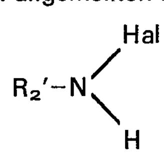 (IX)

in der

Hal ein Chlor- oder Bromatom und

$R_2'$ mit Ausnahme von Wasserstoff die für $R_2$ eingangs erwähnten Bedeutungen besitzt, oder mit dessen Alkalisalz und gegebenenfalls anschliessende Hydrolyse.

Die Umsetzung wird vorzugsweise mit einem Alkalisalz einer Verbindung der allgemeinen Formel IX, z.B. dem Natriumsalz, gegebenenfalls in Gegenwart einer anorganischen Base wie einer Alkalibase in einem Lösungsmittel oder Lösungsmittelgemisch wie Methanol, Methanol/Wasser oder Äthanol zweckmässigerweise bei Temperaturen zwischen 0 und 80° C, vorzugsweise jedoch bei Temperaturen zwischen 5 und 50° C, durchgeführt.

Die gegebenenfalls anschliessende Hydrolyse wird in Gegenwart einer Säure oder Base, vorzugsweise jedoch in Gegenwart einer Base wie Natronlauge, in einem Lösungsmittel oder Lösungsmittelgemisch wie Wasser, Methanol, Wasser/Methanol oder Tetrahydrofuran/Wasser bei Temperaturen bis zur Siedetemperatur des verwendeten Lösungsmittels durchgeführt.

Die erfindungsgemäss erhaltenen Verbindungen der allgemeinen Formel I können gewünschtenfalls anschliessend in ihre Salze mit starken Säuren übergeführt werden. Als Säuren kommen beispielsweise Salzsäure, Schwefelsäure oder Mesitylensulfonsäure in Betracht.

Die als Ausgangsstoffe verwendeten Verbindungen der allgemeinen Formeln II bis IX sind teilweise literaturbekannt bzw. man erhält diese nach üblichen Verfahren.

So sind beispielsweise die als Ausgangsstoffe verwendeten Verbindungen der allgemeinen Formel II Gegenstand der EP-A1-0.003.771 und der Deutschen Patentanmeldung P 30 42 632.5 vom 12. November 1980 bzw. können gemäss den in der EP-A1-0.003.771 beschriebenen Verfahren hergestellt werden.

Die als Ausgangsstoffe verwendeten Verbindungen der allgemeinen Formel III erhält man vorzugsweise durch Umsetzung eines entsprechenden O-Carbonyl- oder O-Sulfonyl-acethydroxam-säureesters mit Schwefelsäure und anschliessende Extraktion nach Zugabe einer Base.

Die als Ausgangsstoffe verwendeten Mercaptoverbindungen der allgemeinen Formel IV erhält man beispielsweise durch Umsetzung eines entsprechenden Thioäthers mit einem Chloramin oder mit O-Mesitylensulfonyl-hydroxylamin.

Eine als Ausgangsstoff verwendete Verbindung der allgemeinen Formel V erhält man beispielsweise durch Oxidation einer entsprechenden Mercaptoverbindung, welche man durch Umsetzung eines entsprechenden Thioäthers mit einem Chloramin und anschliessende Hydrolyse erhält, oder durch Umsetzung einer entsprechenden Mercapto- oder Sulfinylverbindung mit O-Mesitylensulfonyl-hydroxylamin.

Eine als Ausgangsstoff verwendete Verbindung der allgemeinen Formel VII erhält man beispielsweise durch Umsetzung einer entsprechenden Sulfinylverbindung, welche man durch Oxidation einer entsprechenden Thioäthers erhält, durch Umsetzung mit einem entsprechenden O-Mesitylensulfonyl-hydroxylamin und gegebenfalls anschliessende Acylierung.

Eine als Ausgangsstoff verwendete Verbindung der allgemeinen Formel VIII erhält man beispielsweise durch Umsetzung einer entsprechenden Hydroxy- bzw. Mercaptoverbindung mit einem entsprechenden Halogenid in Gegenwart einer Base.

Eine als Ausgangsstoff verwendete Verbindung der allgemeinen Formel IX erhält man beispielsweise durch Umsetzung eines entsprechenden Amids mit einem Hyphohalogenit.

Wie bereits eingangs erwähnt, weisen die neuen Verbindungen der allgemeinen Formel I wertvolle pharmakologische Eigenschaften auf, insbesondere antithrombotische Wirkungen. Diese steigern die Synthese des aggregationshemmenden Prostaglandins $I_2$ (Prostacyclin). Ausserdem stellen die Verbindungen der allgemeinen Formel I, in der n die Zahl 0 darstellt, wertvolle Zwischenprodukte zur Herstellung der neuen Verbindungen der allgemeinen Formel I dar, in der n die Zahl 1 darstellt.

Ferner weisen die Verbindungen der allgemeinen Formel I eine Hemmwirkung auf die Tumormetastasierung auf, diese beruht auf folgenden Eigenschaften der erfindungsgemäss hergestellten Verbindungen:

1. Sie sind *Hemmer der Plättchen-Phosphodiesterase*, die als Hemmer der Tumormetastasierung bekannt sind (H. Gastpar, Thrombosis Research *5*, 277-289 (1974) und K.V. Honn, Science *212*, 1270-1272 (1981)).

2. Die Verbindungen verursachen eine starke Verlängerung der Blutungszeit, d.h. sie hemmen die primäre Hämostase, die erste Anheftung von Thrombozyten am verletzten Gefäss unter Ausbildung eines reinen Plättchenthrombus schon bei einer sehr niedrigen Dosierung. Das ist bei den Verbindungen der Formel I durch eine Einschränkung der Plättchenfunktion allein nicht mehr erklärbar, sondern durch eine erhöhte Prostacylinfreisetzung der Endothelzellen des Gefässes. Eine Bestätigung ist das Unterbleiben der Blutungszeitverlängerung, wenn man die Prostacyclinsynthese der Endothelzelle durch vorher verabfolgte Cyclooxygenasehemmer unterbricht. Die Verbindungen stellen somit eine bisher noch unbekannte, optimale Kombination zweier Wirkprinzipien dar, nämlich erhöhte cAMP-Spiegel durch Stimulation der Bildung (Prostacyclin) und gleichzeitig Hemmung des Abbaus (PDE-Hemmung).

Die so erfassbare Steigerung der Prostacyclin-Aktivität bzw. der Prostacyclinsynthese der Gefässwand ist nach HONN (K.V. Honn, Science *212*, 1270-1272 (1981)) ebenfalls Ursache für die Hemmung der Tumormetastasierung.

Beispielsweise wurden die Verbindungen:

A = 6-(4-Methylsulfoximino-butoxy)-3,4-dihydrocarbostyrilmesitylensulfonat,

B = 6-(4-Phenylsulfoximino-butoxy)-3,4-dihydrocarbostyril,

C = 6-[4-(4-Fluorphenylsulfoximino)-butoxy]-3,4-dihydrocarbostyril,

D = 6-[4-(4-Chlorphenylsulfoximino)-butoxy]-3,4-dihydrocarbostyril,

E = 6-[4-(N-Acetyl-3,4-dichlorphenylsulfoximino)-butoxy]-3,4-dihydrocarbostyril,

F = 6-[4-(N-p-Toluolsulfonyl-3,4-dichlor-

phenylsulfoximino)-butoxy]-3,4-dihydrocarbostyril,

G = 6-[4-(4-Fluorphenylsulfoximino)-butoxy]-carbostyril,

H = 6-[4-(4-Chlorphenylsulfoximino)-butoxy]-carbostyril,

I = 6-[4-(3-Methyl-4-bromphenylsulfoximino)-butoxy]-carbostyril,

K = 6-[4-(4-tert.-Butylphenyl-sulfoximino)-butoxy]-3,4-dihydrocarbostyril,

L = 6-[4-(4-Cyclohexylphenyl-sulfoximino)-butoxy]-3,4-dihydrocarbostyril,

M = 6-[4-(4-tert.-Butylphenyl-sulfoximino)-butoxy]-carbostyril,

N = 6-(4-Cyclohexyl-sulfoximino-butoxy)-carbostyril,

O = 6-[4-(N-Butyryl-3,4-dichlorphenyl-sulfoximino)-butoxy]-3,4-dihydrocarbostyril,

P = 5-[4-(N-Acetyl-4-chlorphenylsulfoximino)-butoxy]-3,3-dimethyl-indolin-2-on,

Q = 6-[4-(N-4-tert.-Benzoyl-3,4-dimethoxyphenyl-sulfoximino)-butoxy]-3,4-dihydrocarbostyril

und

R = 6-[4-(3,4-Dimethoxyphenylsulfoximino)-butoxy]-3,4-dihydrocarbostyril

auf ihre biologischen Eigenschaften wie folgt geprüft:

1. *PDE-Hemmung:*

*Prinzip:*

cAMP wird von Phosphodiesterase (PDE) aus verschiedenen Quellen, so auch aus Blutplättchen, zu AMP hydrolysiert. Diese Hydrolyse wird konzentrationsabhängig von PDE-Hemmern inhibiert.

*Methode:*

Als Phosphodiesterase wird der 10.000 × g-Überstand von Humanplättchen verwendet, welche mit Wasser eingefroren und wieder aufgetaut wurden.

0,3 ml einer Mischung, die 0,1 mol/l Trishydroxy-aminomethan (pH 7.4), 3 mmol/l Magnesiumchlorid, 1 mmol/l AMP, 1 µmol/l $^3$H-cAMP (spez. Aktivität ca. 10 MBq/µmol), PDE sowie die zu untersuchende Substanz bzw. Wasser bei der Kontrolle enthält, werden 15 Minuten bei 37° C inkubiert.

Die Inkubation wird durch Zugabe von 0,5 ml Zinksulfat (0,266 mol/l) und 0,5 ml Bariumhydroxid (0,226 mol) gestoppt, der Niederschlag abzentrifugiert und die im Überstand verbleibende Aktivität des nicht umgesetzten $^3$H-cAMP bestimmt. Aus dem Vergleich der Substanz-Ansätze gegenüber Kontroll-Ansätze wurde die Konzentration für eine 50%ige Hemmwirkung ($IC_{50}$) der jeweiligen Substanz berechnet:

| Substanz | $IC_{50}$ (µmol/l) | Substanz | $IC_{50}$ (µmol/l) |
|---|---|---|---|
| A | 45 | K | 0,24 |
| B | 2,0 | L | 0,052 |
| C | 1,2 | M | 0,21 |
| D | 0,84 | N | 0,68 |
| E | 0,84 | O | 0,53 |
| F | 0,90 | P | 1,8 |
| G | 0,40 | Q | 3,1 |
| H | 0,17 | R | 1,6 |
| I | 0,039 | | |

Die Hemmwirkung auf die Tumormetastasierung lässt sich auch nach Gastpar et al. (siehe Thrombosis Research *5,* 277-289 (1974)), als eine die Tumorzellenembolie verhindernde Wirkung belegen. Hierbei wird die zu untersuchende Substanz vor der Tumorzellentransplantation appliziert und die Überlebensrate der Versuchstiere, beispielsweise von Ratten, gegen Kontrollen bestimmt.

2. *Bestimmung der Verlängerung der Blutungszeit:*

*Vorbemerkung:*

Der menschliche Organismus sowie der Warmblüter besitzt einen sinnvollen Mechanismus, der ihn vor Blutverlusten im Falle von Verletzungen schützen soll. Dieses System besteht aus den Blutplättchen (Thrombozyten), welche mittels ihrer Klebeeigenschaften einen Gefässdefekt rasch „verstopfen" sollen und so die *primäre Hämostase* herbeiführen. Neben diesem reinen cellulären Blutstillungsmechanismus besitzt der Körper ein Blutgerinnungssystem. Bei diesem System werden Plasmafaktoren (Eiweisskörper) in eine wirksame Form gebracht, welche schliesslich das flüssige Plasmafibrinogen zu einem Fibringerinnsel werden lassen. Das System der primären Hämostase, welches im wesentlichen von den Thrombozyten, aber auch von der Prostacyclin-Aktivität der Gefässwand geregelt wird, und das Gerinnungssystem ergänzen sich in dem gemeinsamen Ziel, den Körper vor Blutverlusten wirkungsvoll zu schützen.

Bei manchen Krankheiten kann es auch bei einem intakten Gefässsystem zum Ablaufen von Gerinnungsprozessen sowie zum Verklumpen von Thrombozyten kommen. Die Schwächung des Blutgerinnungssystems durch Cumarine oder Heparin ist bekannt und kann leicht mit Hilfe von bekannten Blutgerinnungstesten gemessen werden, welche unter Präparateeinwirkung eine Verlängerung anzeigen (Plasmarecalcif.-zeit, Quick-Bestimmung, Thrombinzeit etc.).

Da im Falle einer Verletzung die erste rasche Blutstillung durch Haftung und Aggregation der Thrombocyten an der Gefässwand geschieht, lässt sich beim Setzen einer standardisierten Verletzung die Funktion der Thrombozyten bzw. die Prostacyclinaktivität der Gefässwand mit Hilfe der Messung der Blutungszeit gut bestimmen. Die normale Blutungszeit beträgt beim Menschen etwa 1 bis 3 min, setzt aber leistungsfähige und in genügender Zahl vorhandene Thrombozyten voraus. Bei einer normalen Thrombozytenzahl weist also eine

verlängerte Blutungszeit auf eine gestörte Funktion der Thrombocyten und/oder auf eine erhöhte Prospacyclin-Aktivität der Gefässwand hin. Wir finden dies z.B. bei einigen angeborenen Thrombocytenfunktionsstörungen. Will man auf der anderen Seite die Neigung zu spontanem Zusammenballen der Thrombozyten- mit der Folge von Gefässverschlüssen im arteriellen System durch Medikamente verhindern, so muss folglich bei einer erfolgreichen, auf die Thrombozyten oder die Gefässwand wirksamen Therapie die Blutungszeit unter Substanzeinfluss verlängert werden. Wir erwarten also bei einer antithrombotisch wirksamen Substanz eine Verlängerung der Blutungszeit und – da das plasmatische Gerinnungssystem ja nicht berührt wird – eine normale Blutgerinnungszeit.

Literatur: W.D. Keidel: Kurzgefasstes Lehrbuch der Physiologie, Georg Thieme Verlag Stuttgart 1967, Seite 31: Der Blutstillungsvorgang.

Zur Bestimmung der Blutungszeit wurden die zu untersuchenden Substanzen wachen Mäusen in einer Dosis von 2,5 mg/kg p.o. appliziert. Nach einer Stunde wurde von der Schwanzspitze jedes Tieres ca. 0,5 mm abgeschnitten und das austretende Blut in Abständen von 30 Sekunden vorsichtig mit einem Filterpapier abgetupft. Die Zahl der so erhaltenen Bluttropfen ergab ein Mass für die Blutungszeit (5 Tiere pro Versuch). Die folgenden Zahlenangaben bedeuten Prozent-Verlängerung gegenüber Kontrollen:

| Substanz | Verlängerung der Blutungszeit in % nach 1 Stunde | Substanz | Verlängerung der Blutungszeit in % nach 1 Stunde |
|---|---|---|---|
| A | 73 | K | >180 |
| B | >161 | L | >191 |
| C | 125 | M | >191 |
| D | 53 | N | >184 |
| E | >194 | O | >233 |
| F | >212 | P | >169 |
| G | 85 | Q | >158 |
| H | 78 | R | >218 |
| I | >275 | | |

2. *Akute Toxizität:*

Die akute Toxizität der zu untersuchenden Substanzen wurde orientierend an Gruppen von je 10 Mäusen nach oraler Gabe einer Einzeldosis bestimmt (Beobachtungszeit: 14 Tage):

| Substanz | Orientierende akute Toxizität |
|---|---|
| A | >250 mg (0 von 10 Tieren gestorben) |
| B | >250 mg (0 von 10 Tieren gestorben) |
| C | >250 mg (0 von 10 Tieren gestorben) |
| D | >250 mg (0 von 10 Tieren gestorben) |
| E | >1000 mg (0 von 10 Tieren gestorben) |
| F | >250 mg (0 von 10 Tieren gestorben) |
| G | >250 mg (0 von 10 Tieren gestorben) |
| H | >250 mg (0 von 10 Tieren gestorben) |
| I | >250 mg (0 von 10 Tieren gestorben) |

Die erfindungsgemäss hergestellten neuen Verbindungen eignen sich aufgrund ihrer oben erwähnten pharmakologischen Eigenschaften zur Prophylaxe thrombo-embolischer Erkrankungen wie Coronarinfarkt, Cerebralinfarkt, sog. transient ischaemic attacks, Amaurosis fugax, zur Prophylaxe der Arteriosklerose und zur Metastasenprophylaxe.

Die zur Erzielung einer entsprechenden Wirkung erforderlichen Dosierung beträgt zweckmässigerweise 2- bis 4mal täglich 0,1 bis 4 mg/kg Körpergewicht, vorzugsweise 0,2 bis 3 mg/kg Körpergewicht. Hierzu lassen sich die erfindungsgemäss hergestellten Verbindungen der allgemeinen Formel I sowie ihre physiologisch verträglichen Säureadditionssalze mit starken Säuren, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, nichtionische Tenside wie z.B. Polyoxyäthylen-Fettsäureester, Wasser/Polyäthylenglykol, Propylenglykol, Cetylstearylalkohol, Carboxymethylcellulose oder fetthaltige Substanzen wie Hartfett oder deren geeignete Gemische, in übliche galenische Zubereitungen wie Tabletten, Dragées, Kapseln, Pulver, Suspensionen, Tropfen, Ampullen, Säfte oder Zäpfchen einarbeiten.

Die nachfolgenden Beispiele sollen die Erfindung näher erläutern:

*Beispiel 1:*

*6-(4-Phenylsulfoximino-butoxy)-3,4-dihydrocarbostyril*

3,4 g (0,01 mol) 6-(4-Phenylsulfinyl-butoxy)-3,4-dihydrocarbostyril werden bei 45° C in 50 ml Polyphosphorsäure eingerührt. Nachdem praktisch alles gelöst ist, gibt man innerhalb 30 Minuten 0,98 g (0,015 mol) Natriumazid in kleinen Portionen hinzu. Es ist eine schwache Entwicklung von Stickstoffgas zu bemerken. Man rührt die beigefarbene, cremig-schaumige Masse 3 Stunden bei 45-50° C und versetzt dann mit 150 g Eis. Die entstehende trübe Lösung stellt man mit konzentriertem Ammoniak auf pH 8 und extrahiert das ausfallende harzige Produkt mit Chloroform. Der ölige Eindampfrückstand wird aus Essigester umkristallisiert. Man erhält weisse Kristalle.

Schmelzpunkt: 127-129° C.

Ausbeute: 1,6 g (44,6% der Theorie).

*Beispiel 2:*

*6-[4-(3,4-Dichlorphenylsulfoximino)-butoxy]-3,4-dihydrocarbostyril*

24,0 g (0,84 mol) O-Mesitylensulfonyl-acethydroxamsäure-äthyl-ester werden in 35 ml Dioxan gelöst und mit 17 ml 90%iger Schwefelsäure bei 20-23° C innerhalb 20 Minuten tropfenweise unter gutem Rühren versetzt. Man rührt noch 10 Minuten bei der gleichen Temperatur nach, giesst dann in 300 ml Eiswasser ein und extrahiert das

gebildete O-Mesitylensulfonyl-hydroxylamin mit 100 ml Methylenchlorid, wäscht noch zweimal mit Eiswasser und trocknet über Magnesiumsulfat. In die erhaltene Lösung trägt man 12,4 g (0,03 mol) 6-[4-(3,4-Dichlorphenylsulfinyl)-butoxy]-3,4-dihydrocarbostyril ein und rührt 18 Stunden bei Zimmertemperatur. Der kaum noch rührbare Kristallbrei wird mit 120 ml Essigester verdünnt und anschliessend das kristalline 6-[4-(3,4-Dichlorphenylsulfoximino)-butoxy]-3,4-dihydrocarbostyril-mesitylensulfonat abgesaugt. Zur Gewinnung der freien Base suspendiert man in 60 ml Methanol und verrührt mit 17 ml 2 n Natronlauge, wobei alles in Lösung geht. Nach kurzer Zeit fällt ein weisser Niederschlag des 6-[3-(3,4-Dichlorphenylsulfoximino)-butoxy]-3,4-dihydrocarbostyril aus.

Schmelzpunkt: 160-161° C.
Ausbeute: 10,4 g (81,1% der Theorie).

*Beispiel 3:*

*6-[4-(4-tert.-Butylphenylsulfoximino)-butoxy]-3,4-dihydrocarbostyril*

Hergestellt analog Beispiel 2 aus 6-[4-(4-tert.-Butylphenylsulfinyl)-butoxy]-3,4-dihydrocarbostyril und O-Mesitylensulfonylhydroxylamin.
Schmelzpunkt: 201-203° C.
Ausbeute: 44% der Theorie.

*Beispiel 4:*

*6-[4-(3,5-Di-tert.-butyl-4-hydroxy-phenylsulfoximino)-butoxy]-3,4-dihydrocarbostyril*

Hergestellt analog Beispiel 2 aus 6-[4-(3,5-Di-tert.-butyl-4-hydroxyphenylsulfinyl)-butoxy]-3,4-dihydrocarbostyril und O-Mesitylensulfonylhydroxylamin.
Schmelzpunkt: 110-112° C.
Ausbeute: 52% der Theorie.

*Beispiel 5:*

*6-[4-(4-Cyclohexylphenylsulfoximino)-butoxy]-3,4-dihydrocarbostyril*

Hergestellt analog Beispiel 2 aus 6-[4-(4-Cyclohexylphenylsulfinyl)-butoxy]-3,4-dihydrocarbostyril und O-Mesitylensulfonylhydroxylamin.
Schmelzpunkt: 174-176° C.
Ausbeute: 65% der Theorie.

*Beispiel 6:*

*6-[4-(4-Biphenylylsulfoximino)-butoxy]-3,4-dihydrocarbostyril*

Hergestellt analog Beispiel 2 aus 6-[4-(4-Biphenylylsulfinyl)-butoxy]-3,4-dihydrocarbostyril und O-Mesitylensulfonylhydroxylamin.
Schmelzpunkt: 184-186° C.
Ausbeute: 64% der Theorie.

*Beispiel 7:*

*6-[4-Naphthyl-(2)-sulfoximino)-butoxy]-3,4-dihydrocarbostyril*

Hergestellt analog Beispiel 2 aus 6-[4-(Naphthyl-2-sulfinyl)-butoxy]-3,4-dihydrocarbostyril und O-Mesitylensulfonylhydroxylamin.
Schmelzpunkt: 151-152° C.
Ausbeute: 71% der Theorie.

*Beispiel 8:*

*6-[4-(4-Fluorphenylsulfoximino)-butoxy]-3,4-dihydrocarbostyril*

Hergestellt analog Beispiel 1 aus 6-[4-(4-Fluorphenylsulfinyl)-butoxy]-3,4-dihydrocarbostyril und Natriumazid in Polyphosphorsäure.
Schmelzpunkt: 170-173° C.
Ausbeute: 78% der Theorie.

*Beispiel 9:*

*6-[4-(4-Chlorphenylsulfoximino)-butoxy]-3,4-dihydrocarbostyril*

Hergestellt analog Beispiel 2 aus 6-[4-(4-Chlorphenylsulfinyl)-butoxy]-3,4-dihydrocarbostyril und O-Mesitylensulfonylhydroxylamin.
Schmelzpunkt: 150-151° C.
Ausbeute: 60% der Theorie.

*Beispiel 10:*

*6-[4-(3-Methyl-4-brom-phenylsulfoximino)-butoxy]-3,4-dihydrocarbostyril*

Hergestellt analog Beispiel 2 aus 6-[4-(3-Methyl-4-bromphenylsulfinyl)-butoxy]-3,4-dihydrocarbostyril und O-Mesitylensulfonylhydroxylamin.
Schmelzpunkt: 150-152° C.
Ausbeute: 60% der Theorie.

*Beispiel 11:*

*6-[4-(3,5-Dibrom-4-aminophenylsulfoximino)-butoxy]-3,4-dihydrocarbostyril*

Hergestellt analog Beispiel 2 aus 6-[4-(3,4-Dibrom-4-aminophenylsulfinyl)-butoxy]-3,4-dihydrocarbostyril und O-Mesitylensulfonylhydroxylamin.
Schmelzpunkt: 110-113° C.
Ausbeute: 55% der Theorie.

*Beispiel 12:*

*6-(4-Phenylsulfoximino-butoxy)-carbostyril*

Hergestellt analog Beispiel 2 aus 6-(4-Phenylsulfinyl-butoxy)-carbostyril und O-Mesitylensulfonylhydroxylamin.
Schmelzpunkt: 161-162° C.
Ausbeute: 60% der Theorie.

*Beispiel 13:*

*6-[4-(4-tert.-Butylphenylsulfoximino)-butoxy]-carbostyril*

Hergestellt analog Beispiel 1 aus 6-[4-(4-tert.-Butylphenylsulfinyl)-butoxy]-carbostyril und Natriumazid in Polyphosphorsäure.
Schmelzpunkt: 208-210° C.
Ausbeute: 45% der Theorie.

*Beispiel 14*

*6-[4-(3,5-Di-tert.-butyl-4-hydroxy-phenylsulfoximino)-butoxy]-carbostyril*

Hergestellt analog Beispiel 2 aus 6-[4-(3,4-Di-tert.-butyl-4-hydroxy-phenylsulfinyl)-butoxy]-

carbostyril und O-Mesitylensulfonylhydroxylamin.
Schmelzpunkt: 205-207° C.
Ausbeute: 59% der Theorie.

*Beispiel 15:*

*6-[4-(4-Cyclohexylphenylsulfoximino)-butoxy]-carbostyril*

Hergestellt analog Beispiel 1 aus 6-[4-(4-Cyclohexylphenylsulfinyl)-butoxy]-carbostyril und Natriumazid in Polyphosphorsäure.
Schmelzpunkt: 195-197° C.
Ausbeute: 52% der Theorie.

*Beispiel 16:*

*6-[4-(4-Biphenylylsulfoximino)-butoxy]-carbostyril*

Hergestellt analog Beispiel 2 aus 6-[4-(4-Biphenylylsulfinyl)-butoxy]-carbostyril und O-Mesitylensulfonylhydroxylamin.
Schmelzpunkt: 236-238° C.
Ausbeute: 76% der Theorie.

*Beispiel 17:*

*6-(4-Cyclohexylsulfoximino-butoxy)-carbostyril*

Hergestellt analog Beispiel 1 aus 6-(4-Cyclohexylsulfinyl)-butoxy-carbostyril und Natriumazid in Polyphosphorsäure.
Schmelzpunkt: 145-147° C.
Ausbeute: 47% der Theorie.

*Beispiel 18:*

*6-[4-(4-Fluorphenylsulfoximino)-butoxy]-carbostyril*

Hergestellt analog Beispiel 2 aus 6-[4-(4-Fluorphenylsulfinyl)-butoxy]-carbostyril und O-Mesitylensulfonylhydroxylamin.
Schmelzpunkt: 177-179° C.
Ausbeute: 70% der Theorie.

*Beispiel 19:*

*6-[4-(4-Chlorphenylsulfoximino)-butoxy]-carbostyril*

Hergestellt analog Beispiel 2 aus 6-[4-(4-Chlorphenylsulfinyl)-butoxy]-carbostyril und O-Mesitylensulfonylhydroxylamin.
Schmelzpunkt: 201-203° C.
Ausbeute: 79% der Theorie.

*Beispiel 20:*

*6-[4-(4-Bromphenylsulfoximino)-butoxy]-carbostyril*

Hergestellt analog Beispiel 2 aus 6-[4-(4-Bromphenylsulfinyl)-butoxy]-carbostyril und O-Mesitylensulfonylhydroxylamin.
Schmelzpunkt: 211-213° C.
Ausbeute: 63% der Theorie.

*Beispiel 21:*

*6-[4-(3,4-Dichlorphenylsulfoximino)-butoxy]-carbostyril*

Hergestellt analog Beispiel 2 aus 6-[4-(3,4-Dichlorphenylsulfinyl)-butoxy]-carbostyril und O-Mesitylensulfonylhydroxylamin.
Schmelzpunkt: 214-216° C.
Ausbeute: 73% der Theorie.

*Beispiel 22:*

*6-[4-(3-Methyl-4-bromphenylsulfoximino)-butoxy]-carbostyril*

Hergestellt analog Beispiel 1 aus 6-[4-(3-Methyl-4-bromphenylsulfinyl)-butoxy]-carbostyril und Natriumazid in Polyphosphorsäure.
Schmelzpunkt: 193-194° C.
Ausbeute: 54% der Theorie.

*Beispiel 23:*

*6-[4-(2′-Fluor-4-biphenylylsulfoximino)-butoxy]-carbostyril*

Hergestellt analog Beispiel 2 aus 6-[4-(2′-Fluor-4-biphenylylsulfinyl)-butoxy]-carbostyril und O-Mesitylensulfonylhydroxylamin.
Schmelzpunkt: 191-193° C.
Ausbeute: 74% der Theorie.

*Beispiel 24:*

*6-[4-(3,5-Dibrom-4-aminophenylsulfoximino)-butoxy]-carbostyril*

Hergestellt analog Beispiel 2 aus 6-[4-(3,5-Dibrom-4-aminophenylsulfinyl)-butoxy]-carbostyril und O-Mesitylensulfonylhydroxylamin.
Schmelzpunkt: 130-135° C.
Ausbeute: 51% der Theorie.

*Beispiel 25:*

*3,3-Dimethyl-5-[4-(4-methylphenylsulfoximino)-butoxy]-indolinon-2*

Hergestellt analog Beispiel 2 aus 3,3-Dimethyl-5-[4-(4-methylphenylsulfinyl)-butoxy]-indolinon-2 und O-Mesitylensulfonylhydroxylamin.
Schmelzpunkt: 146-147° C.
Ausbeute: 84% der Theorie.

*Beispiel 26:*

*3,3-Dimethyl-5-[4-(4-tert.-butylphenylsulfoximino)-butoxy]-indolinon-2*

Hergestellt analog Beispiel 2 aus 3,3-Dimethyl-5-[4-(4-tert.-butylphenylsulfinyl)-butoxy]-indolinon-2 und O-Mesitylensulfonylhydroxylamin.
Schmelzpunkt: 195-197° C.
Ausbeute: 85% der Theorie.

*Beispiel 27:*

*3,3-Dimethyl-5-[4-(2-methyl-4-tert.-butylphenylsulfoximino)-butoxy]-indolinon-2*

Hergestellt analog Beispiel 2 aus 3,3-Dimethyl-5-[4-(2-methyl-4-tert.-butylphenylsulfinyl)-butoxy]-indolinon-2 und O-Mesitylensulfonylhydroxylamin.
Schmelzpunkt: 149-150° C.
Ausbeute: 26% der Theorie.

*Beispiel 28:*

*3,3-Dimethyl-5-[4-(3,5-di-tert.-butyl-4-hydroxyphenylsulfoximino)-butoxy]-indolinon-2*

Hergestellt analog Beispiel 2 aus 3,3-Dimethyl-5-[4-(3,5-di-tert.-butyl-4-hydroxyphenylsulfoximino)-butoxy]-indolinon-2 und O-Mesitylensulfonylhydroxylamin.
Glasige Substanz. $R_f$-Wert: 0,25 (Kieselgelplatte, Laufmittel: Essigester/Methylenchlorid 1:1).
Ausbeute: 42% der Theorie.

*Beispiel 29:*

*3,3-Dimethyl-5-(4-cyclohexylphenylsulfoximino-butoxy)-indolinon-2*

Hergestellt analog Beispiel 1 aus 3,3-Dimethyl-5-(4-cyclohexylphenylsulfinyl-butoxy)-indolinon-2 und Natriumazid in Polyphosphorsäure.
Schmelzpunkt: 162-163° C.
Ausbeute: 39% der Theorie.

*Beispiel 30:*

*3,3-Dimethyl-5-[4-(2-naphthylsulfoximino)-butoxy]-indolinon-2*

Hergestellt analog Beispiel 2 aus 3,3-Dimethyl-5-[4-(2-naphthyl-sulfinyl)-butoxy]-indolinon-2 und O-Mesitylensulfonylhydroxylamin.
Schmelzpunkt: 120-121° C.
Ausbeute: 64% der Theorie.

*Beispiel 31:*

*3,3-Dimethyl-5-(4-cyclohexylsulfoximino-butoxy)-indolinon-2*

Hergestellt analog Beispiel 2 aus 3,3-Dimethyl-5-(4-cyclohexylsulfinyl-butoxy)-indolinon-2 und O-Mesitylensulfonylhydroxylamin.
Schmelzpunkt: 108-109° C.
Ausbeute: 77% der Theorie.

*Beispiel 32:*

*3,3-Dimethyl-5-(4-benzylsulfoximino-butoxy)-indolinon-2*

Hergestellt analog Beispiel 2 aus 3,3-Dimethyl-5-(4-benzylsulfinyl-butoxy)-indolinon-2 und O-Mesitylensulfonylhydroxylamin.
Schmelzpunkt: 98-99° C.
Ausbeute: 82% der Theorie.

*Beispiel 33:*

*3,3-Dimethyl-5-[4-(4-fluorphenylsulfoximino)-butoxy]-indolinon-2*

Hergestellt analog Beispiel 2 aus 3,3-Dimethyl-5-[4-(4-fluorphenylsulfinyl)-butoxy]-indolinon-2 und O-Mesitylensulfonylhydroxylamin.
Schmelzpunkt: 101-102° C.
Ausbeute: 90% der Theorie.

*Beispiel 34:*

*3,3-Dimethyl-5-[4-(4-chlorphenylsulfoximino)-butoxy]-indolinon-2*

Hergestellt analog Beispiel 2 aus 3,3-Dimethyl-5-[4-(4-chlorphenylsulfinyl)-butoxy]-indolinon-2 und O-Mesitylensulfonylhydroxylamin.
Schmelzpunkt: 136-137° C.
Ausbeute: 76% der Theorie.

*Beispiel 35:*

*3,3-Dimethyl-5-[4-(4-bromphenylsulfoximino)-butoxy]-indolinon-2*

Hergestellt analog Beispiel 2 aus 3,3-Dimethyl-5-[4-(4-bromphenylsulfinyl)-butoxy]-indolinon-2 und O-Mesitylensulfonylhydroxylamin.
Schmelzpunkt: 160-161° C.
Ausbeute: 88% der Theorie.

*Beispiel 36:*

*3,3-Dimethyl-5-[4-(3,4-dichlorphenylsulfoximino)-butoxy]-indolinon-2*

Hergestellt analog Beispiel 2 aus 3,3-Dimethyl-5-[4-(3,4-dichlorphenylsulfinyl)-butoxy]-indolinon-2 und O-Mesitylensulfonylhydroxylamin.
Schmelzpunkt: 147-148° C.
Ausbeute: 68% der Theorie.

*Beispiel 37:*

*3,3-Dimethyl-5-[4-(2,5-dichlorphenylsulfoximino)-butoxy]-indolinon-2*

Hergestellt analog Beispiel 2 aus 3,3-Dimethyl-5-[4-(2,5-dichlorphenylsulfinyl)-butoxy]-indolinon-2 und O-Mesitylensulfonylhydroxylamin.
$R_f$-Wert: 0,3 (Kieselgelplatte, Laufmittel: Essigester/Methylenchlorid 1:1).
Ausbeute: 18% der Theorie.

*Beispiel 38:*

*3,3-Dimethyl-5-[4-(3-methyl-4-brom-phenylsulfoximino)-butoxy]-indolinon-2*

Hergestellt analog Beispiel 2 aus 3,3-Dimethyl-5-[4-(3-methyl-4-brom-phenylsulfinyl)-butoxy]-indolinon-2 und O-Mesitylensulfonylhydroxylamin.
Schmelzpunkt: 131-132° C.
Ausbeute: 84% der Theorie.

*Beispiel 39:*

*3,3-Dimethyl-5-[4-(2'-fluor-4-biphenylylsulfoximino)-butoxy]-indolinon-2*

Hergestellt analog Beispiel 2 aus 3,3-Dimethyl-5-[4-(2'-fluor-4-biphenylylsulfinyl)-butoxy]-indolinon-2 und O-Mesitylensulfonylhydroxylamin.
Schmelzpunkt: 177-178° C.
Ausbeute: 90% der Theorie.

*Beispiel 40:*

*3,3-Dimethyl-5-[4-(3,5-dibrom-4-aminophenylsulfoximino)-butoxy]-indolinon-2*

Hergestellt analog Beispiel 2 aus 3,3-Dimethyl-5-[4-(3,5-dibrom-4-aminophenylsulfinyl)-butoxy]-indolinon-2 und O-Mesitylensulfonylhydroxylamin.
Schmelzpunkt: 202-204° C.
Ausbeute: 76% der Theorie.

*Beispiel 41:*

*3,3-Dimethyl-5-[4-(4-methoxyphenylsulfoximino)-butoxy]-indolinon-2*

Hergestellt analog Beispiel 2 aus 3,3-Dimethyl-5-[4-(4-methoxyphenylsulfinyl)-butoxy]-indolinon und O-Mesitylensulfonylhydroxylamin.
Schmelzpunkt: 140-141° C.
Ausbeute: 71% der Theorie.

*Beispiel 42:*

*3,3-Dimethyl-5-[4-(2-methoxyphenylsulfoximino)-butoxy]-indolinon-2*

Hergestellt analog Beispiel 2 aus 3,3-Dimethyl-5-[4-(2-methoxyphenylsulfinyl)-butoxy]-indolinon-2 und O-Mesitylensulfonylhydroxylamin.
Farblose harzige Substanz. $R_f$-Wert: 0,35 (Kieselgel, Laufmittel: Äthylenchlorid/Äthanol = 9:1).
Ausbeute: 52% der Theorie.

*Beispiel 43:*

*3,3-Dimethyl-5-[4-(3,4-dimethoxyphenylsulfoximino)-butoxy]-indolinon-2*

Hergestellt analog Beispiel 2 aus 3,3-Dimethyl-5-[4-(3,4-dimethoxy-phenylsulfinyl)-butoxy]-indolinon-2 und O-Mesitylensulfonylhydroxylamin.
Schmelzpunkt: 108-109° C.
Ausbeute: 79% der Theorie.

*Beispiel 44:*

*3,3-Dimethyl-5-[4-(6-methoxy-naphth-2-yl-sulfoximino)-butoxy]-indolinon-2*

Hergestellt analog Beispiel 2 aus 3,3-Dimethyl-5-[4-(6-methoxy-naphth-2-yl-sulfinyl)-butoxy]-indolinon-2 und O-Mesitylensulfonylhydroxylamin.
Schmelzpunkt: 174-175° C.
Ausbeute: 88% der Theorie.

*Beispiel 45:*

*6-(4-Methylsulfoximino-butoxy)-3,4-dihydrocarbostyril-mesitylensulfonat*

Hergestellt analog Beispiel 2 aus 6-(4-Methylsulfinyl-butoxy)-3,4-dihydrocarbostyril und O-Mesitylensulfonylhydroxylamin.
Schmelzpunkt: 130-133° C.
Ausbeute: 87% der Theorie.

*Beispiel 46:*

*3,3-Dimethyl-5-(4-phenylsulfoximino-butoxy)-indolinon-2*

Hergestellt analog Beispiel 2 aus 3,3-Dimethyl-5-(4-phenylsulfinyl-butoxy)-indolinon-2 und O-Mesitylensulfonylhydroxylamin.
Schmelzpunkt: 111-112° C.
Ausbeute: 86% der Theorie.

*Beispiel 47:*

*6-[4-(N-Acetyl-3,4-dichlorphenylsulfoximino)-butoxy]-3,4-dihydrocarbostyril*

In einem Gemisch von 70 ml Eisessig und 70 ml Essigsäureanhydrid werden 1,40 g 6-[4-(3,4-Dichlorphenylsulfoximino)-butoxy]-3,4-dihydrocarbostyril suspendiert und 2½ Stunden gerührt. Man versetzt die entstandene Lösung unter weiterem gutem Rühren mit 300 ml Eiswasser. Nach 10 Minuten beginnen sich weisse Kristalle abzuscheiden. Nach einer Stunde saugt man ab, wäscht mit Wasser nach und kristallisiert aus 140 ml Äthanol unter Zusatz von wenig Aktivkohle um. Man erhält eine weisse, kristalline Substanz, welche man im Umlufttrockenschrank bei 80° C trocknet.
Schmelzpunkt: 150-152° C.
Ausbeute: 12,9 g (84% der Theorie).

*Beispiel 48:*

*6-[4-(N-Carbamoyl-3,4-dichlorphenylsulfoximino)-butoxy]-3,4-dihydrocarbostyril*

In 70 ml Eisessig löst man 1,49 g (0,0035 mol) 6-[4-(3,4-Dichlor-phenylsulfoximino)-butoxy]-3,4-dihydrocarbostyril und versetzt mit 2,8 g (0,035 mol) Kaliumcyanat und rührt 3 Stunden bei Zimmertemperatur. Danach versetzt man unter Rühren mit 40 ml Wasser, wobei das zunächst ausfallende Öl durchkristallisiert. Man saugt ab, kristallisiert aus 65 ml Äthanol um und trocknet die weisse, kristalline Substanz im Umlufttrockenschrank bei 50° C.
Schmelzpunkt: 148-150° C.
Ausbeute: 1,2 g (73% der Theorie).

*Beispiel 49:*

*6-[4-(N-Butyril-3,4-dichlorphenylsulfoximino)-butoxy]-3,4-dihydrocarbostyril*

3,0 g (0,007 mol) 6-[4-(3,4-Dichlorphenylsulfoximino)-butoxy]-3,4-dihydrocarbostyril werden in 15 ml Pyridin suspendiert und mit 0,9 g (1,2×0,007 mol) n-Buttersäurechlorid versetzt. Unter Erwärmung auf 40° C entsteht eine hellgelbe Lösung. Man dampft nach weiteren 90 Minuten Stehen im Wasserstrahlvakuum am Rotationsverdampfer zur Trockne ein, nimmt den Rückstand in Methylenchlorid auf und schüttelt zweimal mit 0,5 n Salzsäure und einmal mit Wasser aus. Nach dem Trocknen über Magnesiumsulfat destilliert man das Lösungsmittel am Rotationsverdampfer ab und kristallisiert den Rückstand aus 15 ml Äthanol um. Man erhält farblose Kristalle.
Schmelzpunkt: 133-135° C.
Ausbeute: 2,4 g (69% der Theorie).

*Beispiel 50:*

*6-[4-(N-Pivaloyl-3,4-dichlorphenylsulfoximino)-butoxy]-3,4-dihydrocarbostyril*

Hergestellt analog Beispiel 49 aus 6-[4-(3,4-Dichlorphenylsulfoximino)-butoxy]-3,4-dihydrocarbostyril und Pivalinsäurechlorid.
Schmelzpunkt: 158-160° C.
Ausbeute: 81% der Theorie.

*Beispiel 51*

*6-[4-(N-(2-Methoxyacetyl)-3,4-dichlorphenylsulfoximino)-butoxy]-3,4-dihydrocarbostyril*

Hergestellt analog Beispiel 49 aus 6-[4-(3,4-Dichlorphenylsulfoximino)-butoxy]-3,4-dihydrocarbostyril und 2-Methoxyacetylchlorid.
Schmelzpunkt: 103-105° C.
Ausbeute: 50% der Theorie.

*Beispiel 52:*

*6-[4-(N-Benzoyl-3,4-dichlorphenylsulfoximino)-butoxy]-3,4-dihydrocarbostyril*

Hergestellt analog Beispiel 49 aus 6-[4-(3,4-Dichlorphenylsulfoximino)-butoxy]-3,4-dihydrocarbostyril und Benzoylchlorid.
Schmelzpunkt: 110-111° C.
Ausbeute: 68% der Theorie.

*Beispiel 53:*

*6-[4-(N-(4-Methoxybenzoyl)-3,4-dichlorphenylsulfoximino)-butoxy]-3,4-dihydrocarbostyril*

Hergestellt analog Beispiel 49 aus 6-[4-(3,4-Dichlorphenylsulfoximino)-butoxy]-3,4-dihydrocarbostyril und 4-Methoxybenzoylchlorid.
Schmelzpunkt: 186-188° C.
Ausbeute: 70% der Theorie.

*Beispiel 54:*

*6-[4-(N-Nicotinoyl-3,4-dichlorphenylsulfoximino)-butoxy]-3,4-dihydrocarbostyril*

Hergestellt analog Beispiel 49 aus 6-[4-(3,4-Dichlorphenylsulfoximino)-butoxy]-3,4-dihydrocarbostyril und Nikotinsäurechlorid-hydrochlorid.
Schmelzpunkt: 101-103° C.
Ausbeute: 94% der Theorie.

*Beispiel 55:*

*6-[4-(N-(4-Methylphenylsulfonyl)-3,4-dichlorphenylsulfoximino)-butoxy]-3,4-dihydrocarbostyril*

Hergestellt analog Beispiel 49 aus 6-[4-(3,4-Dichlorphenylsulfoximino)-butoxy]-3,4-dihydrocarbostyril und p-Toluolsulfochlorid.
Schmelzpunkt: 154-156° C.
Ausbeute: 84% der Theorie.

*Beispiel 56:*

*6-[4-(N-(2-Acetoxy-phenylacetyl)-3,4-dichlorphenylsulfoximino)-butoxy]-3,4-dihydrocarbostyril*

Hergestellt aus 6-[4-(3,4-Dichlorphenylsulfoximino)-butoxy]-3,4-dihydrocarbostyril und O-Acetyl-D,L-Mandelsäurechlorid analog Beispiel 49. Nach Reinigung über eine Kieselgelsäule mit Äthylenchlorid erhielt man die Substanz als nicht kristallisierendes, glasiges Harz.
$R_f$-Wert: 0,2 (Kieselgelplatte, Äthylenchlorid).
Ausbeute: 50% der Theorie.

*Beispiel 57:*

*5-[4-(N-Acetyl-3,4-dichlorphenylsulfoximino)-butoxy]-3,3-dimethyl-indolinon-2*

Hergestellt analog Beispiel 47 aus 5-[4-(3,4-Dichlorphenylsulfoximino)-butoxy]-3,3-dimethyl-indolinon-2 und Acetanhydrid.
Schmelzpunkt: 145-146° C.
Ausbeute: 64% der Theorie.

*Beispiel 58:*

*5-[4-(N-Butyryl-3,4-dichlorphenylsulfoximino)-butoxy]-3,3-dimethyl-indolinon-2*

Hergestellt analog Beispiel 49 aus 5-[4-(3,4-Dichlorphenylsulfoximino)-butoxy]-3,3-dimethyl-indolinon-2 und Buttersäurechlorid.
Schmelzpunkt: 120-122° C.
Ausbeute: 81% der Theorie.

*Beispiel 59:*

*5-[4-(N-(2-Methoxy-acetyl)-3,4-dichlorphenylsulfoximino)-butoxy]-indolinon-2*

Hergestellt analog Beispiel 49 aus 3,3-Dimethyl-5-[4-(3,4-dichlorphenylsulfoximino)-butoxy]-indolinon-2 und 2-Methoxyacetylchlorid.
Schmelzpunkt: 126-128° C.
Ausbeute: 72% der Theorie.

*Beispiel 60:*

*5-[4-(N-Äthoxycarbonyl-3,4-dichlorphenylsulfoximino)-butoxy]-3,3-dimethyl-indolinon-2*

Hergestellt analog Beispiel 49 aus 5-[4-(4-Chlorphenylsulfoximino)-butoxy]-3,3-dimethyl-indolinon-2 und Chlorkohlensäureäthylester.
Schmelzpunkt: 102-104° C.
Ausbeute: 79% der Theorie.

*Beispiel 61:*

*5-[4-(N-Acetyl-4-chlorphenylsulfoximino)-butoxy]-3,3-dimethyl-indolinon-2*

Hergestellt analog Beispiel 47 aus 5-[4-(4-Chlorphenylsulfoximino)-butoxy]-3,3-dimethyl-indolinon-2 und Acetanhydrid.
Schmelzpunkt: 138-140° C.
Ausbeute: 75% der Theorie.

*Beispiel 62:*

*5-[4-(N-Butyryl-4-chlorphenylsulfoximino)-butoxy]-3,3-dimethyl-indolinon-2*

Hergestellt analog Beispiel 49 aus 5-[4-(4-Chlorphenylsulfoximino)-butoxy]-3,3-dimethyl-indolinon-2 und Buttersäurechlorid.
Schmelzpunkt: 166-168° C.
Ausbeute: 38% der Theorie.

*Beispiel 63:*

*5-[4-(N-Pivaloyl-4-chlorphenylsulfoximino)-butoxy]-3,3-dimethyl-indolinon-2*

Hergestellt analog Beispiel 49 aus 5-[4-(4-Chlorphenylsulfoximino)-butoxy]-3,3-dimethyl-indolinon-2 und Pivalinsäurechlorid.
Schmelzpunkt: 95-97° C.
Ausbeute: 76% der Theorie.

*Beispiel 64:*

*5-[4-(N-Capryl-4-chlorphenylsulfoximino)-butoxy]-3,3-dimethyl-indolinon-2*

Hergestellt analog Beispiel 49 aus 5-[4-(4-Chlorphenylsulfoximino)-butoxy]-3,3-dimethyl-indolinon-2 und Caprylsäurechlorid. Harz.

$R_f$-Wert: 0,5 (Kieselgelplatte, Essigester/Methylenchlorid = 1:1).
Ausbeute: 95% der Theorie.

*Beispiel 65:*

*5-[4-(N-Carbamoyl-4-chlorphenylsulfoximino)-butoxy]-3,3-dimethyl-indolinon-2*

Hergestellt analog Beispiel 48 aus 5-[4-(4-Chlorphenylsulfoximino)-butoxy]-3,3-dimethyl-indolinon-2 und Kaliumcyanat. Farbloses Harz.
$R_f$-Wert: 0,4 (Kieselgelplatte, Äthylenchlorid/Äthanol = 9:1).
Ausbeute: 75% der Theorie.

*Beispiel 66:*

*5-[4-(N-Dimethylaminocarbonyl-4-chlorphenylsulfoximino)-butoxy]-3,3-dimethyl-indolinon-2*

Hergestellt analog Beispiel 49 aus 5-[4-(4-Chlorphenylsulfoximino)-butoxy]-3,3-dimethyl-indolinon-2 und Dimethylcarbamylchlorid.
Schmelzpunkt: 170-172° C.
Ausbeute: 64% der Theorie.

*Beispiel 67:*

*6-[4-(N-Acetyl-3,4-dimethoxyphenylsulfoximino)-butoxy]-3,4-dihydrocarbostyril*

Hergestellt analog Beispiel 47 aus 6-[4-(3,4-Dimethoxyphenylsulfoximino)-butoxy]-3,4-dihydrocarbostyril und Acetanhydrid.
Schmelzpunkt: 89-92° C.
Ausbeute: 60% der Theorie.

*Beispiel 68:*

*6-[4-(N-Butyryl-3,4-dimethoxyphenylsulfoximino)-butoxy]-3,4-dihydrocarbostyril*

Hergestellt analog Beispiel 49 aus 6-[4-(3,4-Dimethoxyphenylsulfoximino)-butoxy]-3,4-dihydrocarbostyril und Buttersäurechlorid. Glasiges, farbloses Harz. $R_f$-Wert: 0,35 (Kieselgelplatte, Äthylenchlorid/Äthanol = 9:1).
Ausbeute: 41% der Theorie.

*Beispiel 69:*

*6-[4-(N-Benzoyl-3,4-dimethoxyphenylsulfoximino)-butoxy]-3,4-dihydrocarbostyril*

Hergestellt analog Beispiel 49 aus 6-[4-(3,4-Dimethoxyphenylsulfoximino)-butoxy]-3,4-dihydrocarbostyril und Benzoylchlorid.
Schmelzpunkt: 78-80° C.
Ausbeute: 50% der Theorie.

*Beispiel 70:*

*6-[4-(N-(4-Chlorbenzoyl)-3,4-dimethoxyphenylsulfoximino)-butoxy]-3,4-dihydrocarbostyril*

Hergestellt analog Beispiel 49 aus 6-[4-(3,4-Dimethoxyphenylsulfoximino)-butoxy]-3,4-dihydrocarbostyril und 4-Chlor-benzoylchlorid.
Schmelzpunkt: 134-137° C.
Ausbeute: 61% der Theorie.

*Beispiel 71:*

*6-[4-(N-(4-tert.-Butylbenzoyl)-3,4-dimethoxyphenylsulfoximino)-butoxy]-3,4-dihydrocarbostyril*

Hergestellt analog Beispiel 49 aus 6-[4-(3,4-Dimethoxyphenylsulfoximino)-butoxy]-3,4-dihydrocarbostyril und 4-tert.-Butylbenzoylchlorid.
Schmelzpunkt: 98-101° C.
Ausbeute: 87% der Theorie.

*Beispiel 72:*

*6-[4-(N-Nicotinoyl-3,4-dimethoxyphenylsulfoximino)-butoxy]-3,4-dihydrocarbostyril*

Hergestellt analog Beispiel 49 aus 6-[4-(3,4-Dimethoxyphenylsulfoximino)-butoxy]-3,4-dihydrocarbostyril und Nicotinsäurechlorid-hydrochlorid.
Schmelzpunkt: 90-93° C.
Ausbeute: 75% der Theorie.

*Beispiel 73:*

*6-[4-(N-Pentamethylphenylsulfonyl-3,4-dimethoxyphenylsulfoximino)-butoxy]-3,4-dihydrocarbostyril*

Hergestellt analog Beispiel 49 aus 6-[4-(3,4-Dimethoxyphenylsulfoximino)-butoxy]-3,4-dihydrocarbostyril und Pentamethylbenzol-sulfonsäurechlorid.
Schmelzpunkt: 186-188° C.
Ausbeute: 75% der Theorie.

*Beispiel 74:*

*5-[4-(N-Acetyl-3,4-dimethoxyphenylsulfoximino)-butoxy]-3,3-dimethylindolin-2-on*

Hergestellt analog Beispiel 47 aus 5-[4-(3,4-Dimethoxyphenylsulfoximino)-butoxy]-3,3-dimethylindolin-2-on und Acetanhydrid. Glasiges Harz.
$R_f$-Wert: 0,3 (Kieselgelplatte, Äthylenchlorid/Äthanol = 9:1).
Ausbeute: 99% der Theorie.

*Beispiel 75:*

*5-[4-(N-Butyryl-3,4-dimethoxyphenylsulfoximino)-butoxy]-3,3-dimethylindolin-2-on*

Hergestellt analog Beispiel 49 aus 5-[4-(3,4-Dimethoxyphenylsulfoximino)-butoxy]-3,3-dimethylindolin-2-on und Buttersäurechlorid. Harz.
$R_f$-Wert: 0,35 (Kieselgelplatte, Essigester/Methylenchlorid = 1:1).
Ausbeute: 92% der Theorie.

*Beispiel 76:*

*5-[4-(N-Pivaloyl-3,4-dimethoxyphenylsulfoximino)-butoxy]-3,3-dimethyl-indolinon-2*

Hergestellt analog Beispiel 49 aus 5-[4-(3,4-Dimethoxyphenylsulfoximino)-butoxy]-3,3-dimethyl-indolinon-2 und Pivalinsäurechlorid. Harz.
$R_f$-Wert: 0,45 (Kieselgelplatte, Essigester/Methylenchlorid = 1:1).
Ausbeute: 88% der Theorie.

*Beispiel 77:*

*5-[4-(N-Carbamoyl-3,4-dimethoxyphenylsulfoximino)-butoxy]-3,3-dimethylindolinon-2*

Hergestellt analog Beispiel 48 aus 5-[4-(3,4-Dimethoxyphenylsulfoximino)-butoxy]-3,3-dimethyl-indolinon-2 und Kaliumcyanat. Harz.

$R_f$-Wert: 0,25 (Kieselgelplatte, Äthylenchlorid/Äthanol = 9:1).

Ausbeute: 75% der Theorie.

*Beispiel 78:*

*5-[4-(N-Dimethylaminocarbonyl-3,4-dimethoxyphenylsulfoximino)-butoxy]-3,3-dimethylindolinon-2*

Hergestellt analog Beispiel 49 aus 5-[4-(3,4-Dimethoxyphenylsulfoximino)-butoxy]-3,3-dimethyl-indolinon-2 und Dimethylcarbamylchlorid. Harz.

$R_f$-Wert: 0,3 (Kieselgelplatte, Äthylenchlorid/Äthanol = 9:1).

Ausbeute: 98% der Theorie.

*Beispiel 79:*

*5-[4-(N-(4-Chlorbenzoyl)-3,4-dimethoxyphenylsulfoximino)-butoxy]-3,3-dimethyl-indolinon-2*

Hergestellt analog Beispiel 49 aus 5-[4-(3,4-Dimethoxyphenylsulfoximino)-butoxy]-3,3-dimethylindolinon-2 und 4-Chlorbenzoylchlorid.

Schmelzpunkt: 174-177° C.

Ausbeute: 74% der Theorie.

*Beispiel 80:*

*5-[4-(N-Nicotinoyl-3,4-dimethoxyphenylsulfoximino)-butoxy]-3,3-dimethyl-indolinon-2*

Hergestellt analog Beispiel 49 aus 5-[4-(3,4-Dimethoxyphenylsulfoximino)-butoxy]-3,3-dimethyl-indolinon-2 und Nicotinsäurechlorid-hydrochlorid. Harz.

$R_f$-Wert: 0,25 (Kieselgelplatte, Äthylenchlorid/Äthanol = 9:1).

Ausbeute: 76% der Theorie.

*Beispiel 81:*

*6-[4-(N-(2-Naphthoyl)-3,4-dimethoxyphenylsulfoximino)-butoxy]-3,4-dihydrocarbostyril*

Hergestellt analog Beispiel 49 aus 6-[4-(3,4-Dimethoxyphenylsulfoximino)-butoxy]-3,4-dihydrocarbostyril und 2-Naphthoesäurechlorid. Harz.

$R_f$-Wert: 0,45 (Kieselgelplatte, Essigester/Methylenchlorid = 1:1).

Ausbeute: 93% der Theorie.

*Beispiel 82:*

*6-[4-(N-1-Naphthoyl)-3,4-dimethoxyphenylsulfoximino)-butoxy]-3,4-dihydrocarbostyril*

Hergestellt analog Beispiel 49 aus 6-[4-(3,4-Dimethoxyphenylsulfoximino)-butoxy]-3,4-dihydrocarbostyril und 1-Naphthoesäurechlorid. Harz.

$R_f$-Wert: 0,3 (Kieselgelplatte, Essigester/Methylenchlorid = 1:1).

Ausbeute: 83% der Theorie.

*Beispiel 83:*

*6-[4-(N-(2-Thienoyl)-3,4-dimethoxyphenylsulfoximino)-butoxy]-3,4-dihydrocarbostyril*

Hergestellt analog Beispiel 49 aus 6-[4-(3,4-Dimethoxyphenylsulfoximino)-butoxy]-3,4-dihydrocarbostyril und Thiophen-2-carbonsäurechlorid. Harz.

$R_f$-Wert: 0,3 (Kieselgelplatte, Essigester/Methylenchlorid = 1:1).

Ausbeute: 88% der Theorie.

*Beispiel 84:*

*6-[4-(3,4-Dimethoxyphenylsulfoximino)-butoxy]-3,4-dihydrocarbostyril*

Hergestellt analog Beispiel 2 aus 6-[4-(3,4-Dimethoxyphenylsulfinyl)-butoxy]-3,4-dihydrocarbostyril und O-Mesitylensulfonylhydroxylamin.

Schmelzpunkt: 154-156° C.

Ausbeute: 64% der Theorie.

*Beispiel 85:*

*6-[4-(N-Benzoyl-4-fluorphenylsulfoximino)-butoxy]-3,4-dihydrocarbostyril*

Hergestellt analog Beispiel 49 aus 6-[4-(4-Fluorphenylsulfoximino)-butoxy]-3,4-dihydrocarbostyril und Benzoylchlorid.

Schmelzpunkt: 64-68° C.

Ausbeute: 87% der Theorie.

*Beispiel 86:*

*6-[4-(N-(4-Chlorbenzoyl)-4-fluorphenylsulfoximino)-butoxy]-3,4-dihydrocarbostyril*

Hergestellt analog Beispiel 49 aus 6-[4-(4-Fluorphenylsulfoximino)-butoxy]-3,4-dihydrocarbostyril und 4-Chlor-benzoylchlorid.

Schmelzpunkt: 134-138° C.

Ausbeute: 78% der Theorie.

*Beispiel 87:*

*6-[4-(3,4-Dichlorphenylsulfoximino)-butoxy]-3,4-dihydrocarbostyril*

Man löst 145,5 g p-Toluolsulfonsäure unter Rühren in 350 ml Dimethylformamid und rührt 70,1 g 6-[4-(3,4-Dichlorphenylsulfinyl)-butoxy]-3,4-dihydrocarbostyril ein. Dann gibt man 106,7 g O-Mesitylensulfonylhydroxamsäureäthylester hinzu. Die Reaktion ist schwach exotherm, man sorgt durch gelegentliche Kühlung dafür, dass 20° C in dem Reaktionsgemisch nicht überschritten werden. Nach 46 Stunden lässt man unter gutem Rühren 350 ml Wasser zulaufen bis eine erste schwache Trübung auftritt. Nach Zugabe einer Suspension von Impfkristallen beginnt die Kristallisation eines grossen Anteils des Produktes während 30 Minuten unter weiterem Rühren. Zur Vervollständigung rührt man noch weitere 300 ml Wasser ein. Man rührt noch 30 Minuten nach, saugt dann die Kristalle des gebildeten O-Mesitylensulfonsauren Salzes ab und wäscht mit Wasser nach. Der noch wasserfeuchte Nutschenkuchen

wird in 500 ml Methanol suspendiert und man rührt auf einmal 100 ml 2n Natronlauge ein. Die Substanz geht kurzzeitig in Lösung, dann erfolgt aber Kristallisation des freien Sulfoximins. Man rührt noch ½ Stunde bei Raumtemperatur nach, saugt ab und wäscht mit eiskaltem Methanol. Man trocknet im Umlufttrockenschrank bei 80° C.

Schmelzpunkt: 160-162° C.
Ausbeute: 61,7 g (84,9% der Theorie).

*Beispiel 88:*

*6-[4-(3,4-Dichlorphenyl-N-(4-toluolsulfonyl)-sulfoximino)-butoxy]-3,4-dihydrocarbostyril*

200 mg 6-[4-(3,4-Dichlorphenyl-N-(4-toluolsulfonyl)-sulfimino)-butoxy]-3,4-dihydrocarbostyril [hergestellt aus 6-[4-(3,4-Dichlorphenylmercapto)-butoxy]-3,4-dihydrocarbostyril und N-Chlor-4-toluolsulfonamid-Natrium (Chloramin T)] werden in Methanol suspendiert, mit 0,35 ml 2n Natronlauge und 0,06 ml Wasserstoffperoxidlösung (397,4 mg/ml) versetzt und 4 Stunden lang zum Sieden am Rückfluss erhitzt. Zu Beginn des Siedens entsteht eine klare Lösung und nach einer Stunde fallen Kristalle aus. Nach weiterem Erhitzen saugt man die Kristalle heiss ab und trocknet sie an der Luft.

Schmelzpunkt: 155-157° C.
Ausbeute: 59% der Theorie.

*Beispiel 89:*

*6-[3-(3,4-Dichlorphenylsulfoximino)-propoxy]-3,4-dihydrocarbostyril*

Hergestellt analog Beispiel 2 aus 6-[3-(3,4-Dichlorphenylsulfinyl)-propoxy]-3,4-dihydrocarbostyril und O-Mesitylensulfonylhydroxylamin.

Schmelzpunkt: 144-146° C.
Ausbeute: 67% der Theorie.

*Beispiel 90:*

*6-[5-(3,4-Dichlorphenylsulfoximino)-pentoxy]-3,4-dihydrocarbostyril*

Hergestellt analog Beispiel 2 aus 6-[5-(3,4-Dichlorphenylsulfinyl)-pentoxy]-3,4-dihydrocarbostyril und O-Mesitylensulfonylhydroxylamin.

Schmelzpunkt: 154-155° C.
Ausbeute: 29% der Theorie.

*Beispiel 91:*

*6-(3-Äthylsulfoximino-propoxy)-3,4-dihydrocarbostyril*

Hergestellt analog Beispiel 2 aus 6-(3-Äthylsulfinyl-propoxy)-3,4-dihydrocarbostyril und O-Mesitylensulfonylhydroxylamin.

Schmelzpunkt: 107-109° C.
Ausbeute: 61% der Theorie.

*Beispiel 92:*

*6-[4-(N-4-Cyanobenzoyl-3,4-dichlorphenylsulfoximino)-butoxy]-3,4-dihydrocarbostyril*

Hergestellt analog Beispiel 49 aus 6-[4-(3,4-Dichlorphenylsulfoximino)-butoxy]-3,4-dihydrocarbostyril und 4-Cyanobenzoylchlorid.

Schmelzpunkt: 200-202° C.
Ausbeute: 79% der Theorie.

*Beispiel 93:*

*6-[4-(N-2-Thenoyl-3,4-dichlorphenylsulfoximino)-butoxy]-3,4-dihydrocarbostyril*

Hergestellt analog Beispiel 49 aus 6-[4-(3,4-Dichlorphenylsulfoximino)-butoxy]-3,4-dihydrocarbostyril und 2-Thiophencarbonsäurechlorid.

Schmelzpunkt: 100-102° C.
Ausbeute: 61% der Theorie.

*Beispiel 94:*

*6-[4-(N-(+)-Pinanoyl-3,4-dichlorphenylsulfoximino)-butoxy]-3,4-dihydrocarbostyril*

Hergestellt analog Beispiel 49 aus 6-[4-(3,4-Dichlorphenylsulfoximino)-butoxy]-3,4-dihydrocarbostyril und (+)-Pinansäurechlorid.

Schmelzpunkt: 69-74° C.
Ausbeute: 94% der Theorie.

*Beispiel 95:*

*6-[4-(N-(−)-Pinanoyl-3,4-dichlorphenylsulfoximino)-butoxy]-3,4-dihydrocarbostyril*

Hergestellt analog Beispiel 49 aus 6-[4-(3,4-Dichlorphenylsulfoximino)-butoxy]-3,4-dihydrocarbostyril und (−)-Pinansäurechlorid.

Schmelzpunkt: 69-74° C.
Ausbeute: 91% der Theorie.

*Beispiel 96:*

*6-[N-Pentamethylphenylsulfonyl-3,4-dichlorphenylsulfoximino)-butoxy]-3,4-dihydrocarbostyril*

Hergestellt analog Beispiel 49 aus 6-[4-(3,4-Dichlorphenylsulfoximino)-butoxy]-3,4-dihydrocarbostyril und Pentamethylphenylsulfochlorid.

Schmelzpunkt: 149-151° C.
Ausbeute: 83% der Theorie.

*Beispiel 97:*

*6-[4-(N-Methansulfonyl-3,4-dichlorphenylsulfoximino)-butoxy]-3,4-dihydrocarbostyril*

Hergestellt analog Beispiel 49 aus 6-[4-(3,4-Dichlorphenylsulfoximino)-butoxy]-3,4-dihydrocarbostyril und Methansulfonylchlorid.

Schmelzpunkt: 172-174° C.
Ausbeute: 37% der Theorie.

*Beispiel 98:*

*6-[4-(N-Acetyl-4-cyclohexylphenylsulfoximino)-butoxy]-3,4-dihydrocarbostyril*

Hergestellt analog Beispiel 47 aus 6-[4-(4-Cyclohexylphenylsulfoximino)-butoxy]-3,4-dihydrocarbostyril und Acetanhydrid.

Schmelzpunkt: 135-136° C.
Ausbeute: 92% der Theorie.

*Beispiel 99:*

*6-[4-(N-Pivaloyl-4-cyclohexylphenylsulfoximino)-butoxy]-3,4-dihydrocarbostyril*

Hergestellt analog Beispiel 49 aus 6-[4-(4-Cyclohexylphenylsulfoximino)-butoxy]-3,4-dihydrocarbostyril und Pivaloylchlorid.
Schmelzpunkt: 170-172° C.
Ausbeute: 95% der Theorie.

*Beispiel 100:*

*6-[4-(N-Benzoyl-4-cyclohexylphenylsulfoximino)-butoxy]-3,4-dihydrocarbostyril*

Hergestellt analog Beispiel 49 aus 6-[4-(4-Cyclohexylphenylsulfoximino)-butoxy]-3,4-dihydrocarbostyril und Benzoylchlorid.
Schmelzpunkt: 187-189° C.
Ausbeute: 92% der Theorie.

*Beispiel 101:*

*6-[4-(N-Acetyl-4-fluorphenylsulfoximino)-butoxy]-3,4-dihydrocarbostyril*

Hergestellt analog Beispiel 47 aus 6-[4-(4-Fluorphenylsulfoximino)-butoxy]-3,4-dihydrocarbostyril ($R_f$-Wert: 0,60) und Acetanhydrid.
$R_f$-Wert: 0,65 (Kieselgel, Laufmittel: Äthylenchlorid/Äthanol = 85:15.
Ausbeute: 54% der Theorie.

*Beispiel 102:*

*6-[4-(N-Butyroyl-4-fluorphenylsulfoximino)-butoxy]-3,4-dihydrocarbostyril*

Hergestellt analog Beispiel 49 aus 6-[4-(4-Fluorphenylsulfoximino)-butoxy]-3,4-dihydrocarbostyril ($R_f$-Wert: 0,60) und Buttersäurechlorid.
$R_f$-Wert: 0,70 (Kieselgel, Laufmittel: Äthylenchlorid/Äthanol = 85:15).
Ausbeute: 78% der Theorie.

*Beispiel 103:*

*6-[4-(N-2-Methoxyacetyl-4-fluorphenylsulfoximino)-butoxy]-3,4-dihydrocarbostyril*

Hergestellt analog Beispiel 49 aus 6-[4-(4-Fluorphenylsulfoximino)-butoxy]-3,4-dihydrocarbostyril ($R_f$-Wert: 0,60) und 2-Methoxyacetylchlorid.
$R_f$-Wert: 0,66 (Kieselgel, Laufmittel: Äthylenchlorid/Äthanol = 85:15).
Ausbeute: 67% der Theorie.

*Beispiel 104:*

*6-[4-(N-(+)-Pinanoyl-4-fluorphenylsulfoximino)-butoxy]-3,4-dihydrocarbostyril*

Hergestellt aus 6-[4-(4-Fluorphenylsulfoximino)-butoxy]-3,4-dihydrocarbostyril und (+)-Pinansäurechlorid analog Beispiel 49.
Schmelzpunkt: 116-120° C.
Ausbeute: 61% der Theorie.

*Beispiel 105:*

*6-[4-(N-(−)-Pinanoyl-4-fluorphenylsulfoximino)-butoxy]-3,4-dihydrocarbostyril*

Hergestellt analog Beispiel 49 aus 6-[4-(4-Fluorphenylsulfoximino)-butoxy]-3,4-dihydrocarbostyril und (−)-Pinansäurechlorid.
Schmelzpunkt: 122-123° C.
Ausbeute: 45% der Theorie.

*Beispiel 106:*

*6-[4-(N-4-Toluolsulfonyl-4-fluorphenylsulfoximino)-butoxy]-3,4-dihydrocarbostyril*

Hergestellt analog Beispiel 49 aus 6-[4-(4-Fluorphenylsulfoximino)-butoxy]-3,4-dihydrocarbostyril und 4-Toluolsulfochlorid.
Schmelzpunkt: 67-70° C.
Ausbeute: 57% der Theorie.

*Beispiel 107:*

*6-[4-(N-(+)-10-Camphersulfonyl-4-fluorphenylsulfoximino)-butoxy]-3,4-dihydrocarbostyril*

Hergestellt analog Beispiel 49 aus 6-[4-(4-Fluorphenylsulfoximino)-butoxy]-3,4-dihydrocarbostyril und (+)-10-Camphersulfochlorid.
Schmelzpunkt: 81-100° C.
Ausbeute: 61% der Theorie.

*Beispiel 108:*

*6-[4-(N-4-Chlorbenzoyl-4-methylsulfoximino)-butoxy]-3,4-dihydrocarbostyril*

Hergestellt analog Beispiel 49 aus 6-(4-Methylsulfoximino)-butoxy)-3,4-dihydrocarbostyril und 4-Chlorbenzoylchlorid.
Schmelzpunkt: 176-178° C.
Ausbeute: 87% der Theorie.

*Beispiel 109:*

*6-(4-n-Hexylsulfoximino-butoxy)-3,4-dihydrocarbostyril*

Hergestellt analog Biespiel 2 aus 6-(4-n-Hexylsulfinyl-butoxy)-3,4-dihydrocarbostyril und O-Mesitylensulfonylhydroxylamin.
Schmelzpunkt: 111-113° C.
Ausbeute: 59% der Theorie.

*Beispiel 110:*

*6-(N-Acetyl-4-n-hexylsulfoximino-butoxy)-3,4-dihydrocarbostyril*

Hergestellt analog Beispiel 47 aus 6-(4-n-Hexylsulfoximino-butoxy)-3,4-dihydrocarbostyril und Acetanhydrid.
Schmelzpunkt: 119-121° C.
Ausbeute: 90% der Theorie.

*Beispiel 111:*

*6-(N-Benzoyl-4-n-hexylsulfoximino-butoxy)-3,4-dihydrocarbostyril*

Hergestellt analog Beispiel 49 aus 6-(4-n-Hexylsulfoximino-butoxy)-3,4-dihydrocarbostyril und Benzoylchlorid.
Schmelzpunkt: 112-114° C.
Ausbeute: 75% der Theorie.

*Beispiel 112:*

*6-[4-(2-Phenyläthylsulfoximino)-butoxy]-3,4-dihydrocarbostyril*

Hergestellt analog Beispiel 2 aus 6-[4-(2-Phe-

nyläthylsulfinyl)-butoxy]-3,4-dihydrocarbostyril und O-Mesitylenhydroxylamin.
Schmelzpunkt: 158-159° C.
Ausbeute: 69% der Theorie.

*Beispiel 113:*

*5-(4-Phenylsulfoximino-butoxy)-3,4-dihydrocarbostyril*

Hergestellt analog Beispiel 2 aus 5-(4-Phenylsulfinyl-butoxy)-3,4-dihydrocarbostyril und O-Mesitylensulfonylhydroxylamin.
Schmelzpunkt: 159-160° C.
Ausbeute: 76% der Theorie.

*Beispiel 114:*

*5-(N-Acetyl-4-phenylsulfoximino-butoxy)-3,4-dihydrocarbostyril*

Hergestellt analog Beispiel 47 aus 5-(4-Phenylsulfoximino-butoxy)-3,4-dihydrocarbostyril und Acetanhydrid.
Schmelzpunkt: 134-137° C.
Ausbeute: 61% der Theorie.

*Beispiel 115:*

*7-(4-Phenylsulfoximino-butoxy)-3-4-dihydrocarbostyril*

Hergestellt analog Beispiel 87 aus 7-(4-Phenylsulfinyl-butoxy)-3,4-dihydrocarbostyril, O-Mesitylensulfonylhydroxamsäureäthylester und p-Toluolsulfonsäure.
Schmelzpunkt: 137-139° C.
Ausbeute: 81% der Theorie.

*Beispiel 116:*

*7-(N-Acetyl-4-phenylsulfoximino-butoxy)-3,4-dihydrocarbostyril*

Hergestellt analog Beispiel 47 aus 7-(4-Phenylsulfoximino)-butoxy)-3,4-dihydrocarbostyril und Acetanhydrid.
Schmelzpunkt: 111-113° C.
Ausbeute: 78% der Theorie.

*Beispiel 117:*

*8-(4-Phenylsulfoximino-butoxy)-3,4-dihydrocarbostyril*

Hergestellt analog Beispiel 2 aus 8-(4-Phenylsulfinyl-butoxy)-3,4-dihydrocarbostyril und Mesitylensulfonylhydroxylamin.
Schmelzpunkt: 78-80° C.
Ausbeute: 96% der Theorie.

*Beispiel 118:*

*8-(N-Acetyl-4-phenylsulfoximino-butoxy)-3,4-dihydrocarbostyril*

Hergestellt analog Beispiel 47 aus 8-(4-Phenylsulfoximino-butoxy)-3,4-dihydrocarbostyril und Acetanhydrid.
Schmelzpunkt: 116-118° C.
Ausbeute: 82% der Theorie.

*Beispiel 119:*

*6-(3-Äthylsulfoximino-propoxy)-carbostyril*

Hergestellt analog Beispiel 2 aus 6-(3-Äthylsulfinyl-propoxy)-carbostyril und O-Mesitylensulfonylhydroxylamin.
Schmelzpunkt: 166-167° C.
Ausbeute: 80% der Theorie.

*Beispiel 120:*

*6-[4-(N-Benzoyl-4-fluorphenylsulfoximino)-butoxy]-carbostyril*

Hergestellt analog Beispiel 49 aus 6-[4-(4-Fluorphenylsulfoximino)-butoxy]-carbostyril und Benzoylchlorid.
Schmelzpunkt: 137-141° C.
Ausbeute: 34% der Theorie.

*Beispiel 121:*

*3,3-Dimethyl-5-[4-(3,4-dimethylphenylsulfoximino)-butoxy]-indolinon-2*

Hergestellt analog Beispiel 87 aus 3,3-Dimethyl-5-[4-(3,4-dimethylphenylsulfinyl)-butoxy]-indolinon-2, O-Mesitylensulfonylhydroxamsäureäthylester und p-Toluolsulfosäure.
Schmelzpunkt: 153-154° C.
Ausbeute: 77% der Theorie.

*Beispiel 122:*

*3,3-Dimethyl-5-[4-(N-acetyl-3,4-dimethylphenylsulfoximino)-butoxy]-indolinon-2*

Hergestellt analog Beispiel 47 aus 3,3-Dimethyl-5-[4-(3,4-dimethylphenylsulfoximino)-butoxy]-indolinon-2 und Acetanhydrid.
Schmelzpunkt: 150-152° C.
Ausbeute: 78% der Theorie.

*Beispiel 123:*

*3,3-Dimethyl-5-(4-methylsulfoximino-butoxy)-indolinon-2*

Hergestellt nach Beispiel 2 aus 3,3-Dimethyl-5-(4-methylsulfinyl-butoxy)-indolinon-2 und O-Mesitylensulfonylhydroxylamin.
Schmelzpunkt: 123-124° C.
Ausbeute: 98% der Theorie.

*Beispiel 124:*

*3,3-Dimethyl-5-[4-(N-4-chlorphenylaminocarbonyl-methylsulfoximino)-butoxy]-indolinon-2*

Hergestellt aus 3,3-Dimethyl-5-(4-methylsulfoximino-butoxy)-indolinon-2 und 4-Chlorphenylisocyanat in absolutem Dioxan bei Zimmertemperatur während 30 Minuten.
Schmelzpunkt: 175-177° C.
Ausbeute: 89% der Theorie.

*Beispiel 125:*

*3,3-Dimethyl-5-[4-(N-4-toluolsulfonyl-4-methylsulfoximino)-butoxy]-indolinon-2*

Hergestellt analog Beispiel 49 aus 3,3-Dimethyl-5-(4-methylsulfoximino-butoxy)-indolinon-2 ($R_f$-Wert: 0,45) und 4-Toluolsulfochlorid.
$R_f$-Wert: 0,70 (Kieselgel, Laufmittel: Essigester/Methylenchlorid = 1:1).
Ausbeute: 63% der Theorie.

*Beispiel 126:*

*3,3-Dimethyl-5-[4-(N-4-chlorbenzoyl-4-methylsulfoximino)-butoxy]-indolinon-2*

Hergestellt analog Beispiel 49 aus 3,3-Dimethyl-5-(4-methylsulfoximino-butoxy)-indolinon-2 und 4-Chlorbenzoylchlorid.

Schmelzpunkt: 176-178° C.

Ausbeute: 73% der Theorie.

*Beispiel 127:*

*6-[4-(N-Acetyl-3,4-dichlorphenylsulfoximino)-butoxy]-3,4-dihydrocarbostyril*

3,4-Dichlorphenylsulfinyl-4-brombutan wird analog Beispiel 2 mit O-Mesitylensulfonylhydroxylamin umgesetzt zu 3,4-Dichlorphenylsulfoximino-4-brombutan und anschliessend analog Beispiel 47 mit Acetanhydrid in 3,4-Dichlorphenyl-(N-acetyl-sulfoximino)-4-brombutan überführt.

Schmelzpunkt: 170-173° C.

Ausbeute: 98% der Theorie.

163,2 mg (0,001 mol) 6-Hydroxycarbostyril, gelöst in 2 ml Dimethylsulfoxid, werden mit 448 mg (0,0013 mol) 3,4-Dichlorphenyl-(N-acetyl-sulfoximino)-4-brombutan und 276,4 mg (0,002 mol) wasserfreiem Kaliumcarbonat versetzt und 17 Stunden lang bei Zimmertemperatur gerührt. Danach wird portionsweise in kleinen Anteilen mit Wasser versetzt, worauf das Reaktionsprodukt in weissen Nadeln auskristallisiert.

Schmelzpunkt: 149-151° C.

Ausbeute: 267,1 mg (62,5% der Theorie).

*Beispiel 128:*

*6-(4-Methylsulfimino-butoxy)-3,4-dihydrocarbostyril-hydrogensulfat*

Man löst 0,12 g Natrium in 25 ml Methanol, trägt unter Rühren 1,3 g 6-(4-Methylmercapto-butoxy)-3,4-dihydrocarbostyril ein und gibt innerhalb 40 Minuten bei einer Temperatur von 17° C 0,57 g Hydroxylamin-O-sulfonsäure hinzu. Dabei steigt die Temperatur bis auf 22° C an. Man rührt über Nacht weiter, saugt dann von ausgeschiedenem Natriumsulfat ab und fällt durch allmähliches Zusetzen mit Äther eine harzige Substanz aus, welche nach Verreiben mit Äther zu einer etwas hygroskopischen Substanz kristallisiert. Man saugt ab und trocknet im Vakuumexsikkator über Calziumchlorid.

Schmelzpunkt: 75-80° C.

Ausbeute: 0,656 g (20% der Theorie).

*Beispiel 129:*

*6-[4-(3,4-Dichlorphenylsulfimino)-butoxy]-3,4-dihydro-carbostyril-mesitylensulfonat*

Hergestellt analog Beispiel 2 aus 6-[4-(3,4-Dichlorphenylmercapto)-butoxy]-3,4-dihydrocarbostyril und O-Mesitylensulfonylhydroxylamin.

Schmelzpunkt: 82-83° C.

Ausbeute: 49% der Theorie.

*Beispiel 130:*

*6-[4-(3,4-Dimethoxyphenylsulfimino)-butoxy]-3,4-dihydrocarbostyril-mesitylensulfonat*

Hergestellt analog Beispiel 2 aus 6-[4-(3,4-Dimethoxyphenyl-mercapto)-butoxy]-3,4-dihydrocarbostyril und O-Mesitylensulfonylhydroxylamin.

Schmelzpunkt: 140-145° C.

Ausbeute: 83% der Theorie.

*Beispiel 131:*

*6-[4-(2'-Fluor-4-biphenylylsulfimino)-butoxy]-carbostyril-mesitylensulfonat*

Hergestellt analog Beispiel 2 aus 6-[4-(2'-Fluor-4-biphenylyl-mercapto)-butoxy]-carbostyril und O-Mesitylensulfonylhydroxylamin.

Schmelzpunkt: 125-128° C.

Ausbeute: 63% der Theorie.

*Beispiel 132:*

*6-[4-(4-tert.-Butylphenylsulfimino)-butoxy]-carbostyril*

Hergestellt analog Beispiel 2 aus 6-[4-(4-tert.-Butylphenylmercapto)-butoxy]-carbostyril und O-Mesitylensulfonylhydroxylamin.

Schmelzpunkt: 145-150° C.

Ausbeute: 55% der Theorie.

*Beispiel 133:*

*6-[4-(N-4-Toluolsulfonyl-4-fluorphenylsulfimino)-butoxy]-3,4-dihydrocarbostyril*

0,70 g 6-[4-(4-Fluorphenylmercapto)-butoxy]-3,4-dihydrocarbostyril werden in 20 ml Methanol suspendiert und mit einer klaren Lösung von 1,16 g N-Chlor-4-toluolsulfonamid-Natrium versetzt. Nach Rühren über Nacht bei Zimmertemperatur destilliert man das Methanol ab. Der erhaltene Rückstand wird an einer Kieselgelsäule mit Methylenchlorid/Essigester = 1:1 chromatographiert.

Schmelzpunkt: 122-124° C.

Ausbeute: 0,59 g (57% der Theorie).

*Beispiel 134:*

*6-[4-(N-4-Toluolsulfonyl-3,4-dichlorphenylsulfimino)-butoxy]-3,4-dihydrocarbostyril*

Hergestellt analog Beispiel 133 aus 6-[4-(3,4-Dichlorphenylmercapto)-butoxy]-3,4-dihydrocarbostyril und N-Chlor-4-toluolsulfonamid-Natrium.

Schmelzpunkt: 150-152° C.

Ausbeute: 62% der Theorie.

*Beispiel 135:*

*5-[4-(N-4-Toluolsulfonyl-4-bromphenylsulfimino)-butoxy]-3,3-dimethyl-indolinon-2*

Hergestellt analog Beispiel 133 aus 5-[4-(4-Bromphenylmercapto)-butoxy]-3,3-dimethylindolinon-2 und N-Chlor-4-toluolsulfonamid-Natrium.

Schmelzpunkt: 163-164° C.

Ausbeute: 33% der Theorie.

*Beispiel 136:*

*6-[4-(N-4-Toluolsulfonyl-methoxyphenylsulfimino)-butoxy]-3,4-dihydrocarbostyril*

Hergestellt analog Beispiel 133 aus 6-[4-(3-Methoxyphenylmercapto)-butoxy]-3,4-dihydrocarbostyril und N-Chlor-4-toluolsulfonamid-Natrium.

Schmelzpunkt: 110-114° C.

Ausbeute: 38% der Theorie.

*Beispiel 137:*

*6-[4-(N-4-Toluolsulfonyl-phenylsulfimino)-butoxy]-3,4-dihydrocarbostyril*

Hergestellt analog Beispiel 133 aus 6-(4-Phenylmercapto)-butoxy)-3,4-dihydrocarbostyril ($R_f$-Wert = 0,45) und N-Chlor-4-toluolsulfonamid-Natrium.

Schmelzpunkt: 58° C (Sintern).

$R_f$-Wert: 0,37 (Kieselgel, Äthylenchlorid/Äthanol = 9:1).

Ausbeute: 62% der Theorie.

*Beispiel 138:*

*6-[4-(N-4-Toluolsulfonyl-cyclohexylsulfimino-butoxy]-3,4-dihydrocarbostyril*

Hergestellt analog Beispiel 133 aus 6-(4-Cyclohexylmercaptobutoxy)-3,4-dihydrocarbostyril und N-Chlor-4-toluolsulfonamid-Natrium.

Schmelzpunkt: 162-163° C.

Ausbeute: 53% der Theorie.

*Beispiel 139:*

*6-[4-(N-4-Toluolsulfonyl-3,4-dichlorphenylsulfimino)-butoxy]-carbostyril*

Hergestellt analog Beispiel 133 aus 6-[4-(3,4-Dichlorphenylmercapto)-butoxy]-carbostyril ($R_f$-Wert: 0,34) und N-Chlor-4-toluolsulfonamid-Natrium.

$R_f$-Wert: 0,32 (Kieselgelplatte, Äthylenchlorid/Äthanol = 9:1).

Ausbeute: 39% der Theorie.

*Beispiel A:*

*Dragées mit 4 mg 6-[4-(N-Acetyl-3,4-dichlorphenylsulfoximino)-butoxy]-3,4-dihydrocarbostyril*

Zusammensetzung:

1 Dragéekern enthält:

| | |
|---|---|
| Wirksubstanz (1) | 4,0 mg |
| Milchzucker (2) | 27,0 mg |
| Maisstärke (3) | 14,5 mg |
| Polyvinylpyrrolidon (4) | 4,0 mg |
| Magnesiumstearat (5) | 0,5 mg |
| | 50,0 mg |

*Herstellung:*

Die Stoffe 1-3 werden mit einer wässerigen Lösung von 4 gleichmässig befeuchtet, durch 1 mm-Maschenweite gesiebt, getrocknet und erneut durch 1 mm-Maschenweite gesiebt. Nach Zumischen von 5 wird die Mischung zu Dragéekernen verpresst.

Dragéekerne: 5 mm ⌀, bikonvex, rund.

*Dragierung:*

Übliche Zuckerdragierung auf 70 mg Endgewicht.

*Beispiel B:*

*Tabletten mit 8 mg 6-[4-(N-Acetyl-3,4-dichlorphenylsulfoximino)-butoxy]-3,4-dihydrocarbostyril*

1 Tablette enthält:

| | |
|---|---|
| Wirksubstanz | 8,0 mg |
| Milchzucker | 23,0 mg |
| Maisstärke | 14,5 mg |
| Polyvinylpyrrolidon | 4,0 mg |
| Magnesiumstearat | 0,5 mg |
| | 50,0 mg |

*Herstellung:*

Analog den Dragéekernen.

*Tablettenbeschreibung:*

Gewicht: 50 mg

Durchmesser: 5 mm, biplan, beidseitige Facette.

*Beispiel C:*

*Suppositorien zu 25 mg 6-[4-(N-Acetyl-3,4-dichlorphenylsulfoximino)-butoxy]-3,4-dihydrocarbostyril*

1 Zäpfchen enthält:

| | |
|---|---|
| Wirksubstanz | 0,025 g |
| Hartfett (z.B. Witepsol H 19 und Witepsol W 45) | 1,695 g |
| | 1,700 g |

*Herstellung:*

Das Hartfett wird geschmolzen. Bei 38° C wird die gemahlene Wirksubstanz in der Schmelze homogen dispergiert. Es wird auf 35° C abgekühlt und in schwach vorgekühlte Suppositorienformen ausgegossen.

Zäpfchengewicht: 1,7 g

*Beispiel D:*

*Suspension mit 8 mg 6-[4-(N-Acetyl-3,4-dichlorphenylsulfoximino)-butoxy]-3,4-dihydrocarbostyril*

100 ml Suspension enthalten:

| | |
|---|---|
| Wirksubstanz | 0,16 g |
| Carboxymethylcellulose | 0,1 g |
| p-Hydroxybenzoesäuremethylester | 0,05 g |
| p-Hydroxybenzoesäurepropylester | 0,01 g |
| Rohrzucker | 10,0 g |
| Glycerin | 5,0 g |
| Sorbitlösung 70% | 20,0 g |
| Aroma | 0,3 g |
| Wasser dest. | ad 100,0 ml |

*Herstellungsverfahren:*

Dest. Wasser wird auf 70° C erhitzt. Hierin wird unter Rühren p-Hydroxybenzoesäuremethylester und -propylester sowie Glycerin und Carboxymethylcellulose gelöst. Es wird auf Raumtemperatur abgekühlt und unter Rühren der Wirkstoff zugegeben und homogen dispergiert. Nach Zugabe und Lösen des Zuckers, der Sorbitlösung und des Aromas wird die Suspension zur Entlüftung unter Rühren evakuiert.

*Beispiel E:*

*Tabletten mit 100 mg 6-[4-(N-Acetyl-3,4-dichlor-phenylsulfoximino)-butoxy]-3,4-dihydrocarbostyril*

Zusammensetzung:
1 Tablette enthält:

| | |
|---|---|
| Wirkstoff | 100,0 mg |
| Milchzucker | 80,0 mg |
| Maisstärke | 34,0 mg |
| Polyvinylpyrrolidon | 4,0 mg |
| Magnesiumstearat | 2,0 mg |
| | 220,0 mg |

*Herstellungsverfahren:*

Wirkstoff, Milchzucker und Stärke werden gemischt und mit einer wässerigen Lösung des Polyvinylpyrrolidons gleichmässig befeuchtet. Nach Siebung der feuchten Masse (2,0 mm-Maschenweite) und Trocknen im Hordentrockenschrank bei 50° C wird erneut gesiebt (1,5 mm-Maschenweite) und das Schmiermittel zugemischt. Die pressfertige Mischung wird zu Tabletten verarbeitet.

Tablettengewicht: 220 mg

Durchmesser: 10 mm, biplan mit beidseitiger Facette und einseitiger Teilkerbe.

*Beispiel F:*

*Hartgelatine-Kapseln mit 150 mg 6-[4-(N-Acetyl-3,4-dichlorphenylsulfoximino)-butoxy]-3,4-dihydrocarbostyril*

1 Kapsel enthält:

| | |
|---|---|
| Wirkstoff | 150,0 mg |
| Maisstärke getr. | ca. 180,0 mg |
| Milchzucker pulv. | ca. 87,0 mg |
| Magnesiumstearat | 3,0 mg |
| | ca. 320,0 mg |

*Herstellung:*

Der Wirkstoff wird mit den Hilfsstoffen vermengt, durch ein Sieb von 0,75 mm-Maschenweite gegeben und in einem geeigneten Gerät homogen gemischt.

Die Endmischung wird in Hartgelatine-Kapseln der Grösse 1 abgefüllt.

Kapselfüllung: ca. 320 mg

Kapselhülle: Hartgelatine-Kapsel Grösse 1.

*Beispiel G:*

*Suppositorien mit 150 mg 6-[4-(N-Acetyl-3,4-dichlor-phenylsulfoximino)-butoxy]-3,4-dihydrocarbostyril*

1 Zäpfchen enthält:

| | |
|---|---|
| Wirkstoff | 150,0 mg |
| Polyäthylenglykol 1500 | 550,0 mg |
| Polyäthylenglykol 6000 | 460,0 mg |
| Polyoxyäthylensorbitanmonostearat | 840,0 mg |
| | 2000,0 mg |

*Herstellung:*

Nach dem Aufschmelzen der Suppositorienmasse wird der Wirkstoff darin homogen verteilt und die Schmelze in vorgekühlte Formen gegossen.

*Beispiel H:*

*Suspension mit 50 mg 6-[4-(N-Acetyl-3,4-dichlor-phenylsulfoximino)-butoxy]-3,4-dihydrocarbostyril pro 5 ml*

100 ml Suspension enthalten:

| | |
|---|---|
| Wirkstoff | 1,0 g |
| Carboxymethylcellulose-Na-Salz | 0,1 g |
| p-Hydroxybenzoesäuremethylester | 0,05 g |
| p-Hydroxybenzoesäurepropylester | 0,01 g |
| Rohrzucker | 10,0 g |
| Glycerin | 5,0 g |
| Sorbitlösung 70%ig | 20,0 g |
| Aroma | 0,3 g |
| Wasser dest. | ad 100 ml |

*Herstellung:*

Dest. Wasser wird auf 70° C erhitzt. Hierin wird unter Rühren p-Hydroxybenzoesäuremethylester und -propylester wobei Glycerin und Carboxymethylcellulose-Natriumsalz gelöst. Es wird auf Raumtemperatur abgekühlt und unter Rühren der Wirkstoff zugegeben und homogen dispergiert. Nach Zugabe und Lösen des Zuckers, der Sorbitlösung und des Aromas wird die Suspension zur Entlüftung unter Rühren evakuiert.

5 ml Suspension enthalten 50 mg Wirkstoff.

*Beispiel I:*

*Tabletten mit 150 mg 6-[4-(N-p-Toluolsulfonyl-3,4-dichlor-phenyl-sulfoximino)-butoxy]-3,4-dihydrocarbostyril*

Zusammensetzung:
1 Tablette enthält:

| | |
|---|---|
| Wirksubstanz | 150,0 mg |
| Milchzucker pulv. | 89,0 mg |
| Maisstärke | 40,0 mg |
| Kolloidale Kieselgelsäure | 10,0 mg |
| Polyvinylpyrrolidon | 10,0 mg |
| Magnesiumstearat | 1,0 mg |
| | 300,0 mg |

*Herstellung:*

Die mit Milchzucker, Maisstärke und Kieselsäure gemischte Wirksubstanz wird mit einer 20%igen wässerigen Polyvinylpyrrolidonlösung befeuchtet und durch ein Sieb mit 1,5 mm-Maschenweite geschlagen.

Das bei 45° C getrocknete Granulat wird nochmals durch dasselbe Sieb gerieben und mit der angegebenen Menge Magnesiumstearat gemischt. Aus der Mischung werden Tabletten gepresst.

Tablettengewicht: 300 mg

Stempel: 10 mm, flach

*Beispiel K:*

*Dragées mit 75 mg 6-[4-(N-p-Toluolsulfonyl-3,4-dichlor-phenyl-sulfoximino)-butoxy]-3,4-dihydrocarbostyril*

1 Dragéekern enthält:

| | |
|---|---|
| Wirksubstanz | 75,0 mg |
| Calciumphosphat | 93,0 mg |
| Maisstärke | 35,5 mg |
| Polyvinylpyrrolidon | 10,0 mg |
| Hydroxypropylmethylcellulose | 15,0 mg |
| Magnesiumstearat | 1,5 mg |
| | 230,0 mg |

*Herstellung:*

Die Wirksubstanz wird mit Calciumphosphat, Maisstärke, Polyvinylpyrrolidon, Hydroxypropylmethylcellulose und der Hälfte der angegebenen Menge Magnesiumstearat gemischt. Auf einer Tablettiermaschine werden Presslinge mit einem Durchmesser von ca. 13 mm hergestellt, diese werden auf einer geeigneten Maschine durch ein Sieb mit 1,5 mm-Maschenweite gerieben und mit der restlichen Menge Magnesiumstearat vermischt. Dieses Granulat wird auf einer Tablettiermaschine zu Tabletten mit der gewünschten Form gepresst.

Kerngewicht: 230 mg

Stempel: 9 mm, gewölbt.

Die so hergestellten Dragéekerne werden mit einem Film überzogen, der im wesentlichen aus Hydroxypropylmethylcellulose besteht. Die fertigen Filmdragées werden mit Bienenwachs geglänzt.

Dragéegewicht: 245 mg.

Selbstverständlich können alle übrigen Verbindungen der allgemeinen Formel I als Wirkstoffe in den vorstehenden galenischen Zubereitungen eingesetzt werden.

**Patentansprüche** für die Vertragsstaaten BE, CH, DE, FR, IT, LI, LU, NL, SE

1. Sulfimine der allgemeinen Formel:

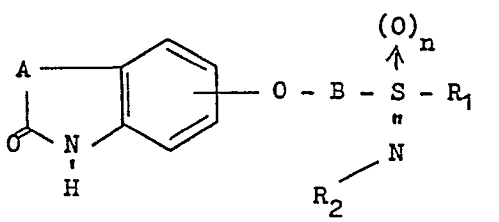

(I)

in der

n die Zahl 0 oder 1,

A eine gegebenenfalls durch eine oder zwei Alkylgruppen mit jeweils 1 bis 3 Kohlenstoffatomen substituierte Methylen-, Vinylen- oder Äthylengruppe,

B eine geradkettige oder verzweigte Alkylengruppe mit 2 bis 6 Kohlenstoffatomen,

$R_1$ eine gegebenenfalls durch eine Phenylgruppe substituierte Alkylgruppe mit 1 bis 3 Kohlenstoffatomen oder eine Phenylgruppe, wobei der Phenylkern jeweils durch eine Alkylgruppe mit 1 bis 4 Kohlenstoffatomen, ein Halogenatom, eine Alkoxygruppe mit 1 bis 3 Kohlenstoffatomen, eine Cyclohexyl-, Phenyl- oder Halogenphenylgruppe substituiert sein kann, eine Alkylgruppe mit 4 bis 7 Kohlenstoffatomen, eine Cycloalkylgruppe mit 3 bis 7 Kohlenstoffatomen, eine durch Alkylgruppen mit 1 bis 4 Kohlenstoffatomen, Alkoxygruppen mit 1 bis 3 Kohlenstoffatomen und/oder Halogenatome di- oder trisubstituierte Phenyl- oder disubstituierte Hydroxy- oder Aminophenylgruppe, wobei die Substituenten des Phenylkerns gleich oder verschieden sein können, eine gegebenenfalls durch eine Alkoxygruppe mit 1 bis 3 Kohlenstoffatomen substituierte Naphthylgruppe oder eine Pyridylgruppe und

$R_2$ ein Wasserstoffatom, eine gegebenenfalls durch eine Methoxygruppe substituierte Alkanoylgruppe mit 1 bis 8 Kohlenstoffatomen, eine gegebenenfalls durch ein Halogenatom, eine Cyangruppe oder eine Alkylgruppe mit 1 bis 4 Kohlenstoffatomen substituierte Benzoyl- oder Phenylsulfonylgruppe, eine Alkoxycarbonylgruppe mit insgesamt 2 bis 4 Kohlenstoffatomen, eine gegebenenfalls durch eine Chlorphenylgruppe, durch eine oder zwei Methylgruppen substituierte Aminocarbonylgruppe, eine Naphthoyl-, Pinanoyl-, Camphersulfonyl-, Pentamethyl-phenylsulfonyl-, Pyridinoyl- oder Thenoylgruppe bedeuten, und deren Salze mit starken Säuren.

2. Physiologisch verträgliche Säureadditionssalze der Verbindungen gemäss Anspruch 1 mit starken physiologisch verträglichen Säuren.

3. Sulfimine der allgemeinen Formel I gemäss Anspruch 1, in der

$R_2$ wie im Anspruch 1 definiert ist,

n die Zahl 0 oder 1,

A eine Dimethylmethylen-, Vinylen- oder Äthylengruppe,

B eine geradkettige Alkylengruppe mit 3 bis 5 Kohlenstoffatomen und

$R_1$ eine Alkylgruppe mit 1 bis 6 Kohlenstoffatomen, eine Benzyl-, Phenyläthyl-, Cyclohexyl-, Naphthyl-, Methoxynaphthyl- oder Pyridylgruppe, eine gegebenenfalls durch eine Alkylgruppe mit 1 bis 4 Kohlenstoffatomen, eine Methoxy-, Cyclohexyl-, Phenyl- oder Fluorphenylgruppe, ein Fluor-, Chlor- oder Bromatom substituierte Phenylgruppe, eine durch Alkylgruppen mit 1 bis 4 Kohlenstoffatomen, Methoxygruppen, Chlor- und/oder Bromatome disubstituierte Phenylgruppe, wobei die Substituenten des Phenylkerns gleich oder verschieden sein können, oder eine durch zwei Chlor- oder Bromatome, zwei Methoxygruppen oder zwei Alkylgruppen mit jeweils 1 bis 4 Kohlenstoffatomen substituierte Aminophenyl-, Hydroxyphenyl- oder Methoxyphenylgruppe bedeuten.

4. Sulfimine der allgemeinen Formel I gemäss Anspruch 1, in der

n die Zahl 1,

A eine Dimethylmethylen-, Vinylen- oder Äthylengruppe,

B eine n-Butylengruppe,

$R_1$ eine Methyl- oder Methoxynaphthylgruppe, eine gegebenenfalls durch eine Methoxygruppe, ein Fluor- oder Chloratom substituierte Phenylgruppe, eine durch Chlor- oder Bromatome substituierte Phenylgruppe, eine Methyl-bromphenyl-, 4-Amino-3,5-dibrom-phenyl- oder Di-tert.-butyl-hydroxy-phenylgruppe und

$R_2$ ein Wasserstoffatom, eine Alkanoylgruppe mit 1 bis 3 Kohlenstoffatomen oder eine gegebenenfalls durch eine Alkylgruppe mit 1 bis 4 Kohlenstoffatomen substituierten Benzoyl- oder Phenylsulfonylgruppe bedeuten.

5. Sulfimine gemäss Anspruch 1 der allgemeinen Formel:

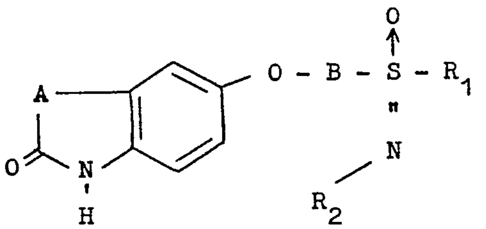
(Ia)

in der

A die Äthylen- oder Vinylengruppe,

B die n-Butylengruppe,

$R_1$ die Methyl-, Phenyl-, 4-Fluorphenyl-, 4-Chlorphenyl-, 3,4-Dichlorphenyl- oder 3-Methyl-4-bromphenylgruppe und

$R_2$ ein Wasserstoffatom, die Acetyl- oder p-Toluolsulfonylgruppe bedeuten.

6. 6-[4-(N-Acetyl-3,4-dichlorphenylsulfoximino)-butoxy]-3,4-dihydrocarbostyril.

7. 6-[4-(3-Methyl-4-bromphenylsulfoximino)-butoxy]-carbostyril.

8. Arzneimittel, enthaltend eine Verbindung gemäss den Ansprüchen 1 bis 7 neben einem oder mehreren inerten Trägerstoffen oder Verdünngsmitteln.

9. Verwendung einer Verbindung gemäss den Ansprüchen 1 bis 7 zur Herstellung eines Arzneimittels zur Bekämpfung thrombo-embolischer Erkrankung, zur Prophylaxe der Arteriosklerose und zur Metastasenprophylaxe.

10. Verfahren zur Herstellung der Verbindungen gemäss den Ansprüchen 1 bis 7, dadurch gekennzeichnet, dass

a) zur Herstellung einer Verbindung der allgemeinen Formel I, in der n die Zahl 1 und $R_2$ ein Wasserstoffatom darstellen, ein Sulfoxid der allgemeinen Formel:

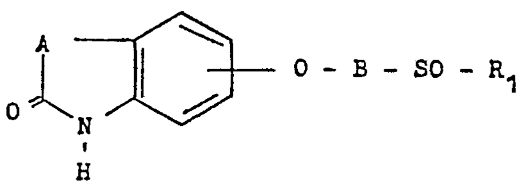
(II)

in der

A, B und $R_1$ wie in den Ansprüchen 1 bis 7 definiert sind, mit gegebenenfalls im Reaktionsgemisch gebildeter Stickstoffwasserstoffsäure umgesetzt wird oder

b) zur Herstellung einer Verbindung der allgemeinen Formel I, in der $R_2$ ein Wasserstoffatom darstellt, eine Verbindung der allgemeinen Formel:

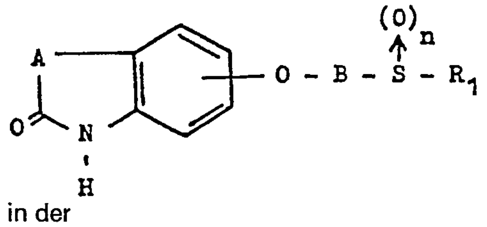
(IIa)

in der

A, B, $R_1$ und n wie in den Ansprüchen 1 bis 7 definiert sind, mit einer Verbindung der allgemeinen Formel:

$$H_2NO-X-R_3 \qquad (III)$$

in der

X eine Carbonyl- oder Sulfonylgruppe und

$R_3$ eine in o-Stellung disubstituierte Arylgruppe wie eine 2,4,6,Trimethyl- oder Triisopropylphenylgruppe oder auch eine Hydroxygruppe darstellt, falls X die Sulfonylgruppe bedeutet, umgesetzt wird oder

c) zur Herstellung einer Verbindung der allgemeinen Formel I, in der n die Zahl 1 darstellt, eine Mercaptoverbindung der allgemeinen Formel:

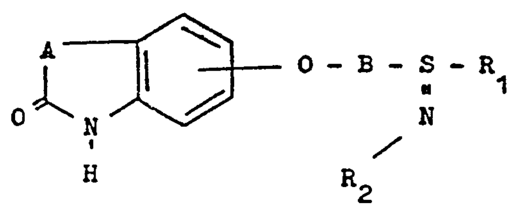
(IV)

in der

A, B, $R_1$ und $R_2$ wie in den Ansprüchen 1 bis 7 definiert sind, oxidiert wird oder

d) zur Herstellung einer Verbindung der allgemeinen Formel I, in der $R_2$ kein Wasserstoffatom darstellt, eine Verbindung der allgemeinen Formel:

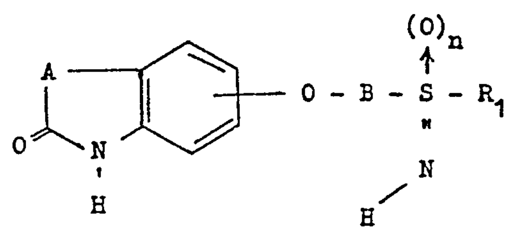
(V)

in der

n, A, B und $R_1$ wie in den Ansprüchen 1 bis 7 definiert sind, acyliert wird oder

e) eine Hydroxyverbindung der allgemeinen Formel:

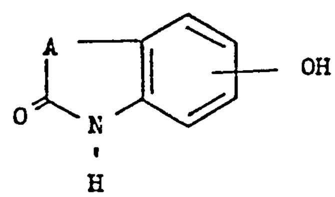
(VI)

in der

A wie in den Ansprüchen 1 bis 7 definiert ist, oder deren Salze mit anorganischen oder tertiären organischen Basen mit einer Verbindung der allgemeinen Formel:

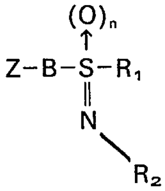
(VII)

in der

n, $R_1$, $R_2$ und B wie in den Ansprüchen 1 bis 7 definiert sind und

Z eine nukleophile Austrittsgruppe darstellt, umgesetzt wird oder

f) zur Herstellung einer Verbindung der allgemeinen Formel I, in der n die Zahl 0 darstellt, ein Thioäther der allgemeinen Formel:

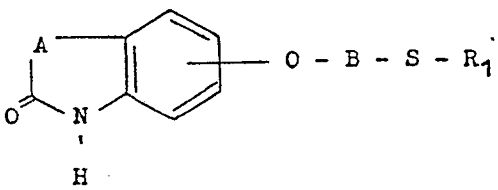
(VIII)

in der

A, B, und $R_1$ wie in den Ansprüchen 1 bis 7 definiert sind, mit einem Amid der allgemeinen Formel:

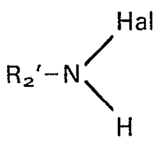
(IX)

in der

Hal ein Chlor- oder Bromatom und

$R_2'$ mit Ausnahme von Wasserstoff die für $R_2$ in den Ansprüchen 1 bis 7 erwähnten Bedeutungen besitzt, oder mit dessen Alkalisalz umgesetzt und das so erhaltene Reaktionsprodukt gegebenenfalls anschliessend hydrolysiert wird

und gewünschtenfalls anschliessend eine erfindungsgemäss erhaltene Verbindung der allgemeinen Formel I mit einer starken Säure in ihr Salz oder in ihr physiologisch verträgliches Salz übergeführt wird.

**Patentansprüche** für den Vertragsstaat: AT

1. Verfahren zur Herstellung von neuen Sulfiminen der allgemeinen Formel:

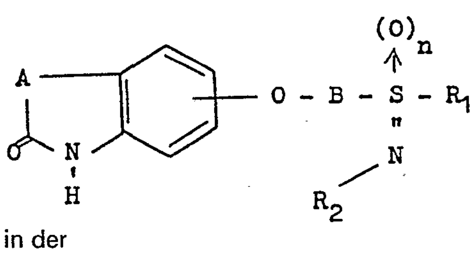
(I)

in der

n die Zahl 0 oder 1,

A eine gegebenenfalls durch eine oder zwei Alkylgruppen mit jeweils 1 bis 3 Kohlenstoffatomen substituierte Methylen-, Vinylen- oder Äthylengruppe,

B eine geradkettige oder verzweigte Alkylengruppe mit 2 bis 6 Kohlenstoffatomen,

$R_1$ eine gegebenenfalls durch eine Phenylgruppe substituierte Alkylgruppe mit 1 bis 3 Kohlenstoffatomen oder eine Phenylgruppe, wobei der Phenylkern jeweils durch eine Alkylgruppe mit 1 bis 4 Kohlenstoffatomen, ein Halogenatom, eine Alkoxygruppe mit 1 bis 3 Kohlenstoffatomen, eine Cyclohexyl-, Phenyl- oder Halogenphenylgruppe substituiert sein kann, eine Alkylgruppe mit 4 bis 7 Kohlenstoffatomen, eine Cycloalkylgruppe mit 3 bis 7 Kohlenstoffatomen, eine durch Alkylgruppen mit 1 bis 4 Kohlenstoffatomen, Alkoxygruppen mit 1 bis 3 Kohlenstoffatomen und/oder Halogenatome di- oder trisubstituierte Phenyl- oder disubstituierte Hydroxy- oder Aminophenylgruppe, wobei die Substituenten des Phenylkerns gleich oder verschieden sein können, eine gegebenenfalls durch eine Alkoxygruppe mit 1 bis 3 Kohlenstoffatomen substituierte Naphthylgruppe oder eine Pyridylgruppe und

$R_2$ ein Wasserstoffatom, eine gegebenenfalls durch eine Methoxygruppe substituierte Alkanoylgruppe mit 1 bis 8 Kohlenstoffatomen, eine gegebenenfalls durch ein Halogenatom, eine Cyangruppe oder eine Alkylgruppe mit 1 bis 4 Kohlenstoffatomen substituierte Benzoyl- oder Phenylsulfonylgruppe, eine Alkoxycarbonylgruppe mit insgesamt 2 bis 4 Kohlenstoffatomen, eine gegebenenfalls durch eine Chlorphenylgruppe, durch eine oder zwei Methylgruppen substituierte Aminocarbonylgruppe, eine Naphthoyl-, Pinanoyl-, Camphersulfonyl-, Pentamethyl-phenylsulfonyl-, Pyridinoyl- oder Thenoylgruppe bedeuten, und deren Salze mit starken Säuren, dadurch gekennzeichnet, dass

a) zur Herstellung einer Verbindung der allgemeinen Formel I, in der n die Zahl 1 und $R_2$ ein Wasserstoffatom darstellen, ein Sulfoxid der allgemeinen Formel:

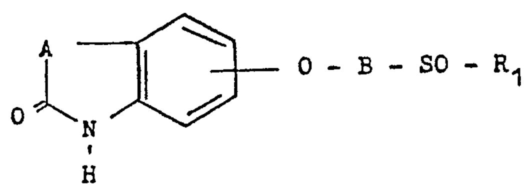
(II)

in der

A, B und $R_1$ wie eingangs definiert sind, mit gegebenenfalls im Reaktionsgemisch gebildeter Stickstoffwasserstoffsäure umgesetzt wird oder

b) zur Herstellung einer Verbindung der allgemeinen Formel I, in der $R_2$ ein Wasserstoffatom darstellt, eine Verbindung der allgemeinen Formel:

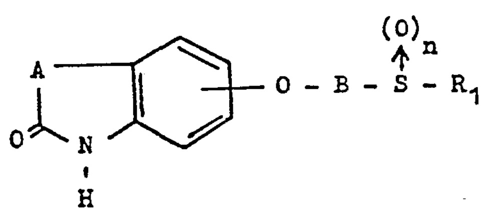
(IIa)

in der

A, B, $R_1$ und n wie eingangs definiert sind, mit einer Verbindung der allgemeinen Formel:

$$H_2NO-X-R_3 \qquad (III)$$

in der

X eine Carbonyl- oder Sulfonylgruppe und

$R_3$ eine in o-Stellung disubstituierte Arylgruppe wie eine 2,4,6-Trimethylphenyl- oder Triisopropylphenylgruppe oder auch eine Hydroxygruppe darstellt, falls X die Sulfonylgruppe bedeutet, umgesetzt wird oder

c) zur Herstellung einer Verbindung der allgemeinen Formel I, in der n die Zahl 1 darstellt, eine Mercaptoverbindung der allgemeinen Formel:

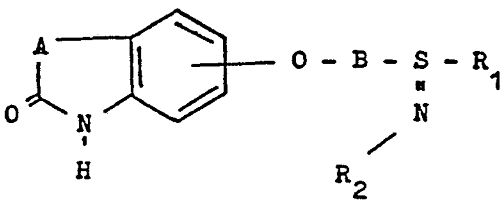 (IV)

in der

A, B, $R_1$ und $R_2$ wie eingangs definiert sind, oxidiert wird oder

d) zur Herstellung einer Verbindung der allgemeinen Formel I, in der $R_2$ kein Wasserstoffatom darstellt, eine Verbindung der allgemeinen Formel:

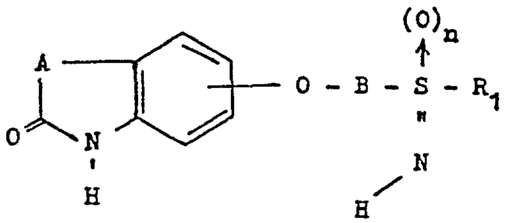 (V)

in der

n, A, B und $R_1$ wie eingangs definiert sind, acyliert wird oder

e) eine Hydroxyverbindung der allgemeinen Formel:

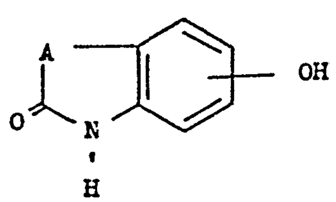 (VI)

in der

A wie eingangs definiert ist, oder deren Salze mit anorganischen oder tertiären organischen Basen mit einer Verbindung der allgemeinen Formel:

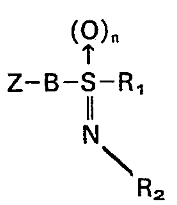 (VII)

in der

n, $R_1$, $R_2$ und B wie eingangs definiert sind und

Z eine nukleophile Austrittsgruppe darstellt, umgesetzt wird oder

f) zur Herstellung einer Verbindung der allgemeinen Formel I, in der n die Zahl 0 darstellt, ein Thioäther der allgemeinen Formel:

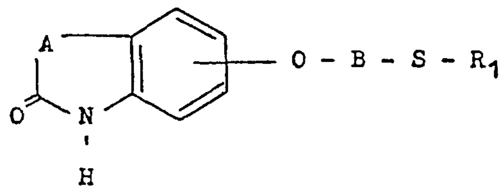 (VIII)

in der

A, B und $R_1$ wie eingangs definiert sind, mit einem Amid der allgemeinen Formel:

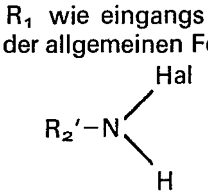 (IX)

in der

Hal ein Chlor- oder Bromatom und

$R_2'$ mit Ausnahme von Wasserstoff die für $R_2$ eingangs erwähnten Bedeutungen besitzt, oder mit dessen Alkalisalz umgesetzt und das so erhaltene Reaktionsprodukt gegebenenfalls anschliessend hydrolysiert wird

und gewünschtenfalls anschliessend eine erfindungsgemäss erhaltene Verbindung der allgemeinen Formel I mit einer starken Säure in ihr Salz oder in ihr physiologisch verträgliches Salz übergeführt wird.

2. Verfahren gemäss Anspruch 1 zur Herstellung von neuen Sulfoximinen der allgemeinen Formel:

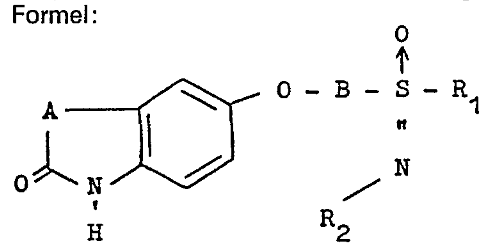 (Ia)

in der

A, B, $R_1$ und $R_2$ wie im Anspruch 1 definiert sind, dadurch gekennzeichnet, dass

a) zur Herstellung einer Verbindung der allgemeinen Formel Ia, in der $R_2$ ein Wasserstoffatom darstellt, ein Sulfoxid der allgemeinen Formel:

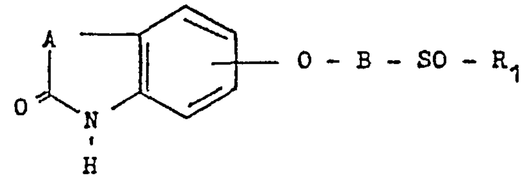 (II)

in der

A, B, $R_1$ wie im Anspruch 1 definiert sind, mit gegebenenfalls im Reaktionsgemisch gebildeter Stickstoffwasserstoffsäure umgesetzt wird oder

b) zur Herstellung einer Verbindung der allgemeinen Formel Ia, in der $R_2$ ein Wasserstoffatom darstellt, ein Sulfoxid der allgemeinen Formel:

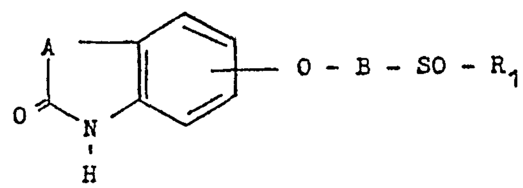 (II)

in der

A, B und $R_1$ wie im Anspruch 1 definiert sind, mit einer Verbindung der allgemeinen Formel:

$$H_2NO-X-R_{3a} \quad \text{(IIIa)}$$

in der

X wie im Anspruch 1 definiert ist und

$R_{3a}$ eine in o-Stellung disubstituierte Arylgruppe wie eine 2,4,6-Trimethyl- oder Triisopropylphenylgruppe darstellt, umgesetzt wird oder

c) eine Mercaptoverbindung der allgemeinen Formel:

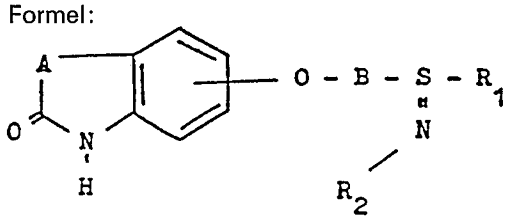 (IV)

in der

A, B, $R_1$ und $R_2$ wie im Anspruch 1 definiert sind, oxidiert wird oder

d) zur Herstellung einer Verbindung der allgemeinen Formel I, in der $R_2$ kein Wasserstoffatom darstellt, eine Verbindung der allgemeinen Formel:

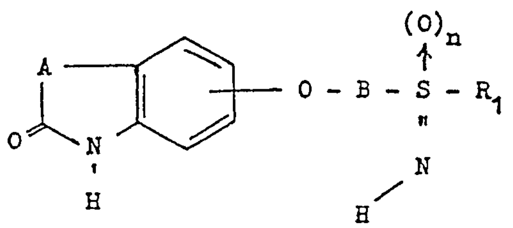 (V)

in der

A, B und $R_1$ wie im Anspruch 1 definiert sind, acyliert wird.

Priorität: 25.07.1981 (P 31 29 444.8).

3. Verfahren nach den Ansprüchen 1 und 2, dadurch gekennzeichnet, dass die Umsetzung in einem Lösungsmittel durchgeführt wird.

4. Verfahren nach den Ansprüchen 1a, 2a und 3, dadurch gekennzeichnet, dass die Umsetzung bei Temperaturen zwischen 0 und 40° C durchgeführt wird.

5. Verfahren nach den Ansprüchen 1b, 2b und 3, dadurch gekennzeichnet, dass die die Umsetzung bei Temperaturen zwsichen 0 und 50° C durchgeführt wird.

6. Verfahren nach den Ansprüchen 1c, 2c und 3, dadurch gekennzeichnet, dass die Oxidation mit einem Äquivalent des eingesetzten Oxidationsmittels und bei Temperaturen zwischen −80 und 100° C durchgeführt wird.

7. Verfahren nach den Ansprüchen 1d, 2d und 3, dadurch gekennzeichnet, dass die Umsetzung bei Temperaturen zwischen −25 und 100° C durchgeführt wird.

8. Verfahren nach den Ansprüchen 1e, 1f und 3, dadurch gekennzeichnet, dass die Umsetzung in Gegenwart einer Alkalibase durchgeführt wird.

9. Verfahren nach den Ansprüchen 1e, 3 und 8, dadurch gekennzeichnet, dass die Umsetzung bei Temperaturen zwischen 0° C und der Siedetemperatur des verwendeten Lösungsmittels durchgeführt wird.

10. Verfahren nach den Ansprüchen 1f, 3 und 8, dadurch gekennzeichnet, dass die Umsetzung bei Temperaturen zwischen 0 und 90° C durchgeführt wird.

**Claims** for the Contracting States BE, CH, DE, FR, IT, LI, LU, NL, SE

1. Sulphimines of general formula:

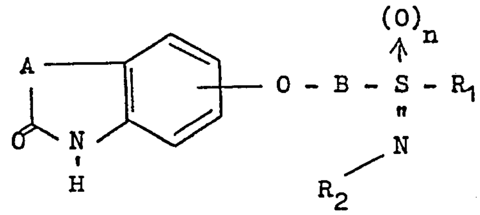 (I)

wherein

n represents the number 0 or 1,

A represents a methylene, vinylene or ethylene group optionally substituted by one or two alkyl groups each having 1 to 3 carbon atoms,

B represents a straight-chained or branched alkylene group with 2 to 6 carbon atoms,

$R_1$ represents an alkyl group with 1 to 3 carbon atoms optionally substituted by a phenyl group, or a phenyl group, whilst the phenyl nucleus may be substituted by an alkyl group with 1 to 4 carbon atoms, a halogen atom, an alkoxy group with 1 to 3 carbon atoms, a cyclohexyl, phenyl or halophenyl group, an alkyl group with 4 to 7 carbon atoms, a cycloalkyl group with 3 to 7 carbon atoms, a phenyl group di- or tri-substituted by alkyl groups with 1 to 4 carbon atoms or by alkoxy groups with 1 to 3 carbon atoms and/or by halogen atoms, or a hydroxy- or aminophenyl group disubstituted by alkyl groups with 1 to 4 carbon atoms or by alkoxy groups with 1 to 3 carbon atoms and/or by halogen atoms, whilst the substituents of the phenyl nucleus may be identical or different, or a naphthyl group optionally substituted by an alkoxy group with 1 to 3 carbon atoms, or a pyridyl group, and

$R_2$ represents a hydrogen atom, an alkanoyl group with 1 to 8 carbon atoms optionally substituted by a methoxy group, a benzoyl or phenylsulphonyl group optionally substituted by a halogen atom or by a cyano group or by an alkyl group with 1 to 4 carbon atoms, an alkoxycarbonyl group with a total of 2 to 4 carbon atoms, an aminocarbonyl group optionally substituted by a chlorophenyl group or by 1 or 2 methyl groups, or a naphthoyl, pinanoyl, camphorsulphonyl, pentamethylphenylsulphonyl, pyridinoyl group or thenoyl group, and the salts thereof with strong acids.

2. Physiologically acceptable acid addition salts of the compounds as claimed in claim 1 with strong physiologically acceptable acids.

3. Sulphimines of general formula I as claimed in claim 1, wherein

$R_2$ is defined as in claim 1,

n represents the number 0 or 1,

A represents a dimethylmethylene, vinylene or ethylene group,

B represents a straight-chained alkylene group with 3 to 5 carbon atoms, and

$R_1$ represents an alkyl group with 1 to 6 carbon atoms, a benzyl, phenylethyl, cyclohexyl, naphthyl, methoxy-naphthyl or pyridyl group, a phenyl group optionally substituted by an alkyl group with 1 to 4 carbon atoms or by a methoxy, cyclohexyl, phenyl or fluorophenyl group or by a fluorine, chlorine or bromine atom, a phenyl group disubstituted by alkyl groups, chlorine and/or bromine atoms, whilst the substituents of the phenyl nucleus may be the same or different, or an aminophenyl, hydroxyphenyl or methoxyphenyl group substituted by two chlorine or bromine atoms, two methoxy groups or two alkyl groups each having from 1 to 4 carbon atoms.

4. Sulphimines of general formula I as claimed in claim 1, wherein

n represents the number 1,

A represents a dimethylmethylene, vinylene or ethylene group,

B represents an n-butylene group,

$R_1$ represents a methyl or methoxynaphthyl group, a phenyl group optionally substituted by a methoxy group or by a fluorine or chlorine atom, a phenyl group substituted by chlorine or bromine atoms, or a methylbromophenyl, 4-amino-3,5-dibromophenyl or di-tert.-butylhydroxy-phenyl group, and

$R_2$ represents a hydrogen atom, an alkanoyl group with 1 to 3 carbon atoms or a benzoyl or phenyl-sulphonyl group optionally substituted by an alkyl group with 1 to 4 carbon atoms.

5. Sulphimines as claimed in claim 1 of general formula:

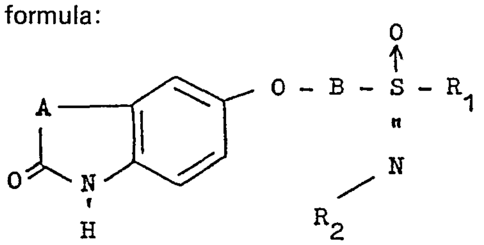
(Ia)

wherein

A represents the ethylene or vinylene group,

B represents the n-butylene group,

$R_1$ represents the methyl, phenyl, 4-fluorophenyl, 4-chlorophenyl, 3,4-dichlorophenyl or 3-methyl-4-bromophenyl group, and

$R_2$ represents a hydrogen atom, the acetyl or p-toluenesulphonyl group.

6. 6-[4-(N-acetyl-3,4-dichlorophenylsulphoximino)-butoxyl]-3,4-dihydrocarbostyrene.

7. 6-[4-(3-methyl-4-bromophenylsulphoximino)-butoxy]-carbostyrene.

8. Pharmaceutical compositions containing a compound as claimed in claims 1 to 7 together with one or more inert carriers or diluents.

9. Use of a compound as claimed in claims 1 to 7 for the preparation of a pharmaceutical composition for combating thrombo-embolic diseases, for the prophylaxis of arteriosclerosis and for the prophylaxis of metastasis.

10. Process for the preparation of the compounds as claimed in claims 1 to 7, characterised in that:

(*a*) to prepare a compound of general formula I wherein n represents the number 1 and $R_2$ represents a hydrogen atom, a sulphoxide of general formula:

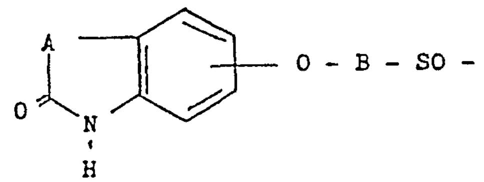
(II)

wherein

A, B and $R_1$ are defined as in claims 1 to 7, is reacted with hydrazoic acid optionally formed in the reaction mixture, or

(*b*) to prepare a compound of general formula I wherein $R_2$ represents a hydrogen atom, a compound of general formula:

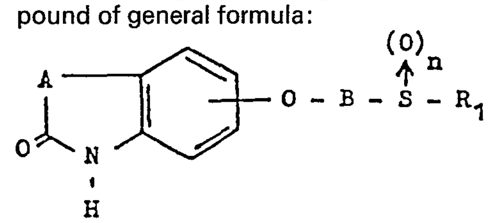
(IIa)

wherein

A, B, $R_1$ and n are defined as in claims 1 to 7, is reacted with a compound of general formula:

$$H_2NO-X-R_3 \qquad (III)$$

wherein

X represents a carbonyl or sulphony group and

$R_3$ represents an aryl group disubstituted in the o-position, such as a 2,4,6-trimethyl- or triisopropylphenyl group or a hydroxy group, if X represents the sulphonyl group, or

(*c*) to prepare a compound of general formula I wherein n represents the number 1, a mercapto compound of general formula:

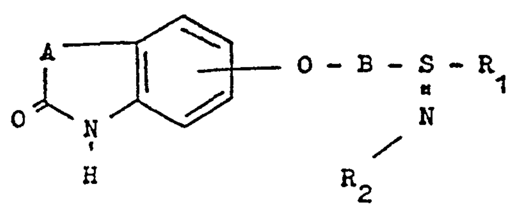
(IV)

wherein

A, B, $R_1$ and $R_2$ are defined as in claims 1 to 7, is oxidised, or

(*d*) to prepare a compound of general formula I wherein $R_2$ does not represent a hydrogen atom, a compound of general formula:

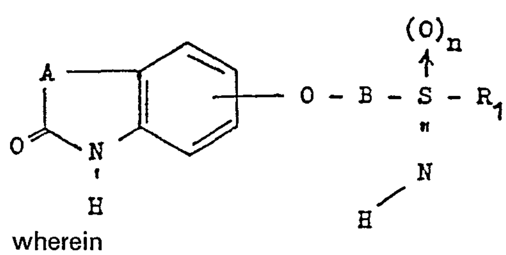
(V)

wherein

n, A, B and $R_1$ are defined as in claims 1 to 7, is acylated, or

(*e*) a hydroxy compound of general formula:

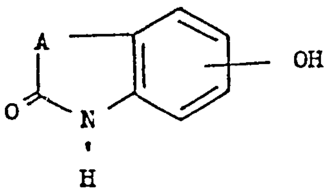 (VI)

wherein

A is defined as in claims 1 to 7, or the salts thereof with inorganic or tertiary organic bases, is reacted with a compound of general formula:

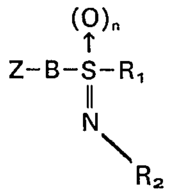 (VII)

wherein

n, $R_1$, $R_2$ and B are defined as in claims 1 to 7 and Z represents a nucleophilic leaving group, or

(*f*) to prepare a compound of general formula I wherein n represents the number 0, a thioether of general formula:

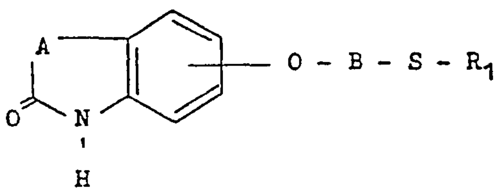 (VIII)

wherein

A, B and $R_1$ are defined as in claims 1 to 7, is reacted with an amide of general formula:

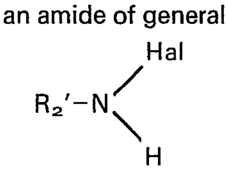 (IX)

wherein

Hal represents a chlorine or bromine atom and

$R_2'$ has the meanings given in claims 1 to 7 for $R_2$, with the exception of hydrogen, or with an alkali salt thereof, and the reaction product thus obtained is subsequently hydrolysed, if required, and if desired a compound of general formula I obtained according to the invention is subsequently converted with a strong acid into a salt thereof or a physiologically acceptable salt thereof.

**Claims** for the Contracting State: AT

1. Process for the preparation of new sulphimines of general formula:

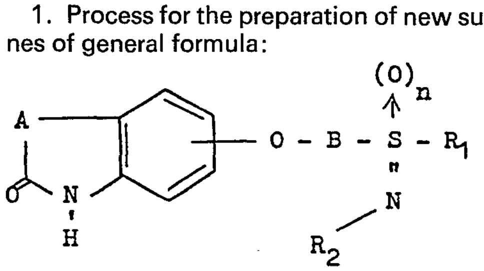 (I)

wherein

n represents the number 0 or 1,

A represents a methylene, vinylene or ethylene group optionally substituted by one or two alkyl groups each having 1 to 3 carbon atoms,

B represents a straight-chained or branched alkylene group with 2 to 6 carbon atoms,

$R_1$ represents an alkyl group with 1 to 3 carbon atoms optionally substituted by a phenyl group, or a phenyl group, whilst the phenyl nucleus may be substituted by an alkyl group with 1 to 4 carbon atoms, a halogen atom, an alkoxy group with 1 to 3 carbon atoms, a cyclohexyl, phenyl or halophenyl group, an alkyl group with 4 to 7 carbon atoms, a cycloalkyl group with 3 to 7 carbon atoms, a phenyl group di- or tri-substituted by alkyl groups with 1 to 4 carbon atoms or by alkoxy groups with 1 to 3 carbon atoms and/or by halogen atoms, or a hydroxy- or aminophenyl group disubstituted by alkyl groups with 1 to 4 carbon atoms or by alkoxy groups with 1 to 3 carbon atoms and/or by halogen atoms, whilst the substituents of the phenyl nucleus may be identical or different, or a naphthyl group optionally substituted by an alkoxy group with 1 to 3 carbon atoms, or a pyridyl group, and

$R_2$ represents a hydrogen atom, an alkanoyl group with 1 to 8 carbon atoms optionally substituted by a methoxy group, a benzoyl or phenylsulphonyl group optionally substituted by a halogen atom or by a cyano group or by an alkyl group with 1 to 4 carbon atoms, an alkoxycarbonyl group with a total of 2 to 4 carbon atoms, an aminocarbonyl group optionally substituted by a chlorophenyl group or by 1 or 2 methyl groups, or a naphthoyl, pinanoyl, camphorsulphonyl, pentamethylphenylsulphonyl, pyridinoyl group or thenoyl group, and the salts thereof with strong acids, characterised in that:

(*a*) to prepare a compound of general formula I wherein n represents the number 1 and $R_2$ represents a hydrogen atom, a sulphoxide of general formula:

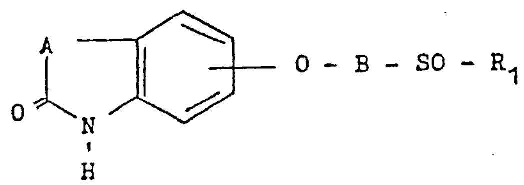 (II)

wherein

A, B and $R_1$ are as hereinbefore defined, is reacted with hydrazoic acid optionally formed in the reaction mixture, or

(*b*) to prepare a compound of general formula I wherein $R_2$ represents a hydrogen atom, a compound of general formula:

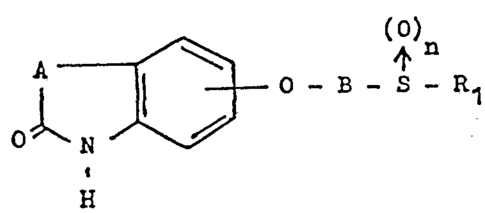 (IIa)

wherein

A, B, $R_1$ and n are as hereinbefore defined, is reacted with a compound of general formula:

$$H_2NO-X-R_3 \quad (III)$$

wherein

X represents a carbonyl or sulphonyl group and

$R_3$ represents an aryl group disubstituted in the o-position, such as a 2,4,6-trimethyl or triisopropylphenyl group or a hydroxy group, if X represents the sulphonyl group, or

(*c*) to prepare a compound of general formula I wherein n represents the number 1, a mercapto compound of general formula:

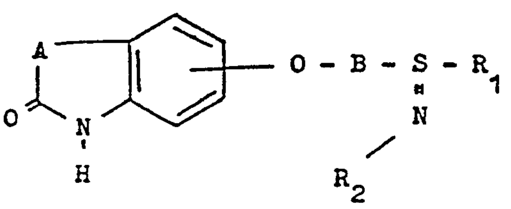
(IV)

wherein

A, B, $R_1$ and $R_2$ are as hereinbefore defined, is oxidised, or

(*d*) to prepare a compound of general formula I wherein $R_2$ does not represent a hydrogen atom, a compound of general formula:

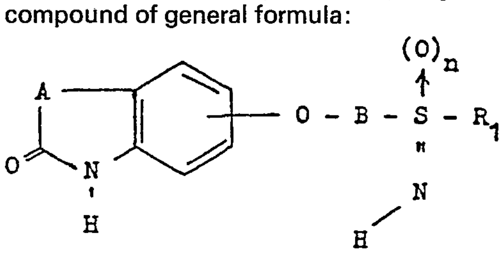
(V)

wherein

n, A, B and $R_1$ are as hereinbefore defined, is acylated, or

(*e*) a hydroxy compound of general formula:

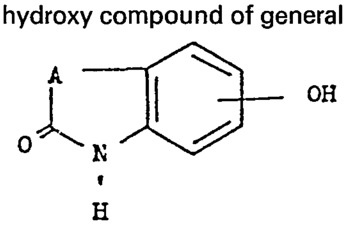
(VI)

wherein

A is as hereinbefore defined, or the salts thereof with inorganic or tertiary organic bases, is reacted with a compound of general formula:

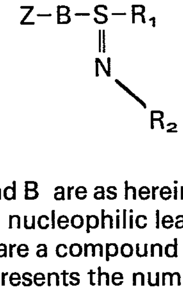
(VII)

wherein

n, $R_1$, $R_2$ and B are as hereinbefore defined and Z represents a nucleophilic leaving group, or

(*f*) to prepare a compound of general formula I wherein n represents the number 0, a thioether of general formula:

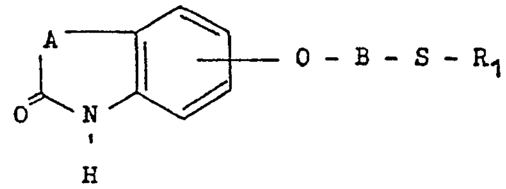
(VIII)

wherein

A, B and $R_1$ are as hereinbefore defined, is reacted with an amide of general formula:

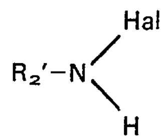
(IX)

wherein

Hal represents a chlorine or bromine atom, and

$R_2'$ has the meanings given in claims 1 to 7 for $R_2$, with the exception of hydrogen, or with an alkali salt thereof, and the reaction product thus obtained is subsequently hydrolysed, if required, and if desired a compound of general formula I obtained according to the invention is subsequently converted with a strong acid into a salt thereof or a physiologically acceptable salt thereof.

2. Process as claimed in claim 1 for preparing new sulphoximines of general formula:

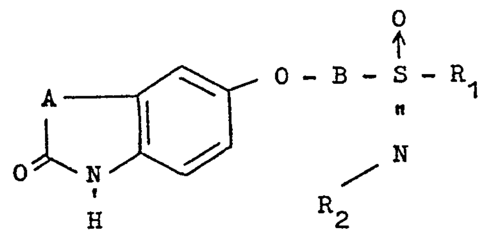
(Ia)

wherein

A, B, $R_1$ and $R_2$ are defined as in claim 1, characterised in that:

(*a*) to prepare a compound of general formula Ia wherein $R_2$ represents a hydrogen atom, a sulphoxide of general formula:

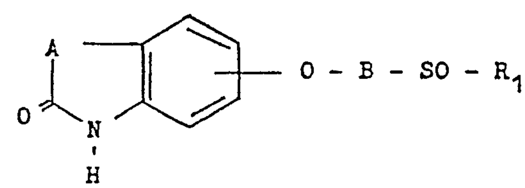
(II)

wherein

A, B and $R_1$ are defined as in claim 1, is reacted with hydrazoic acid optionally formed in the reaction mixture, or

*b*) to prepare a compound of general formula Ia wherein $R_2$ represents a hydrogen atom, a sulphoxide of general formula:

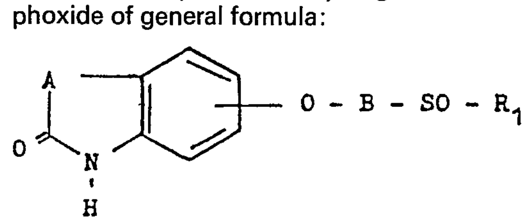
(II)

wherein

A, B and $R_1$ are defined as in claim 1 is reacted

with a compound of general formula:

$$H_2NO{-}X{-}R_{3a} \qquad \text{(IIIa)}$$

wherein

X is defined as in claim 1 and

$R_{3a}$ represents an aryl group disubstituted in the o-position, such as a 2,4,6-trimethyl- or triisopropylphenyl group, or

(*c*) a mercapto compound of general formula:

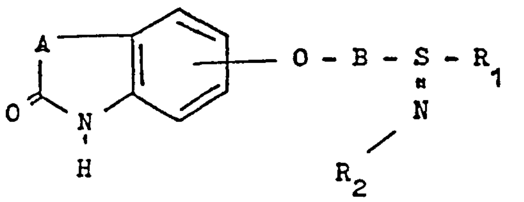

(IV)

wherein

A, B, $R_1$ and $R_2$ are defined as in claim 1, is oxidised, or

(*d*) to prepare a compound of general formula I wherein $R_2$ does not represent a hydrogen atom, a compound of general formula:

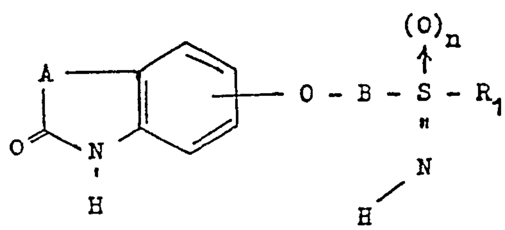

(V)

wherein

A, B and $R_1$ are defined as in claim 1 is acylated.

Priority: 25.07.1981 (P 31 29 444.8)

3. Process as claimed in claims 1 and 2, characterised in that the reaction is carried out in a solvent.

4. Process as claimed in claims 1a, 2a and 3, characterised in that the reaction is carried out at temperatures of between 0 and 40° C.

5. Process as claimed in claims 1b, 2b and 3, characterised in that the reaction is carried out at temperatures of between 0 and 50° C.

6. Process as claimed in claims 1c, 2c and 3, characterised in that the oxidation is effected with one equivalent of the oxidising agent used and at temperatures of between −80 and 100° C.

7. Process as claimed in claims 1d, 2d and 3, characterised in that the reaction is effected at temperatures of between −25 and 100° C.

8. Process as claimed in claims 1e, 1f and 3, characterised in that the reaction is effected in the presence of an alkali base.

9. Process as claimed in claims 1e, 3 and 8, characterised in that the reaction is effected at temperatures of between 0° C and the boiling temperature of the solvent used.

10. Process as claimed in claims 1f, 3 and 8, characterised in that the reaction is carried out at temperatures of between 0 and 80° C.

**Revendications** pour les Etats contractants BE, CH, DE, FR, IT, LI, LU, NL, SE

1. Sulfimines de formule générale:

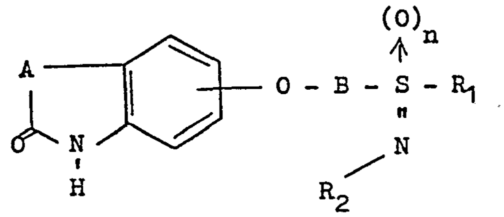

(I)

dans laquelle:

n représente le nombre 0 ou 1,

A représente un groupe méthylène, vinylène ou éthylène éventuellement substitué par un ou deux groupes alcoyle avec chacun 1 à 3 atomes de carbone,

B représente un groupe alcoylène rectiligne ou ramifié avec 2 à 6 atomes de carbone,

$R_1$ représente un groupe alcoyle avec 1 à 3 atomes de carbone éventuellement substitué par un groupe phényle, ou représente un groupe phényle, le noyau phényle pouvant dans chaque cas être substitué par un groupe alcoyle avec 1 à 4 atomes de carbone, un atome d'halogène, un groupe alcoxy avec 1 à 3 atomes de carbone, un groupe cyclohexyle, phényle ou halogénophényle, ou représente un groupe alcoyle avec 4 à 7 atomes de carbone, un groupe cycloalcoyle avec 3 à 7 atomes de carbone, un groupe phényle di- ou trisubstitué ou hydroxy- ou aminophényle disubstitué par des groupes alcoyle avec 1 à 4 atomes de carbone, des groupes alcoxy avec 1 à 3 atomes de carbone et/ou des atomes d'halogène, les substituants du noyau phényle pouvant être identiques ou différents, ou représente un groupe naphtyle éventuellement substitué par un groupe alcoxy avec 1 à 3 atomes de carbone, ou représente un groupe pyridyle, et

$R_2$ représente un atome d'hydrogène, un groupe alcanoyle avec 1 à 8 atomes de carbone éventuellement substitué par un groupe méthoxy, un groupe benzoyle ou phénylsulfonyle éventuellement substitué par un atome d'halogène, un groupe cyano ou un groupe alcoyle avec 1 à 4 atomes de carbone, ou représente un groupe alcoxy carbonyle avec en tout 2 à 4 atomes de carbone, un groupe aminocarbonyle éventuellement substitué par un groupe chlorophényle, par un ou deux groupes méthyle, ou représente un groupe naphtoyle, pinanoyle, camphosulfonyle, pentaméthyl-phénylsulfonyle, pyridinoyle ou thénoyle, et leurs sels avec des acides forts.

2. Sels d'addition d'acides physiologiquement supportables des composés selon la revendication 1 avec des acides forts physiologiquement supportables.

3. Sulfimines de formule générale I selon la revendication 1, dans laquelle:

$R_2$ est défini comme dans la revendication 1,

n représente le nombre 0 ou 1,

A représente un groupe diméthylméthylène, vinylène ou éthylène,

B représente un groupe alcoylène rectiligne avec 3 à 5 atomes de carbone, et

$R_1$ représente un groupe alcoyle avec 1 à 6 atomes de carbone, un groupe benzyle, phényléthyle, cyclohexyle, naphtyle, méthoxynaphtyle ou pyridyle, un groupe phényle éventuellement substitué par un groupe alcoyle avec 1 à 4 atomes de carbone, un groupe méthoxy, cyclohexyle, phényle ou fluorophényle, un atome de fluor, de chlore ou de brome, ou représente un groupe phényle disubstitué par des groupes alcoyle avec 1 à 4 atomes de carbone, des groupes méthoxy, des atomes de chlore et/ou de brome, les substituants du noyau phényle pouvant être identiques ou différents, ou représente un groupe aminophényle, hydroxyphényle ou méthoxyphényle substitué par deux atomes de chlore ou de brome, deux groupes méthoxy ou deux groupes alcoyle avec chacun 1 à 4 atomes de carbone.

4. Sulfimines de formule générale I selon la revendication 1, dans laquelle:

n représente le nombre 1,

A représente un groupe diméthylméthylène, vinylène ou éthylène,

B représente un groupe n-butylène,

$R_1$ représente un groupe méthyl- ou méthoxynaphtyle, un groupe phényle éventuellement substitué par un groupe méthoxy, un atome de fluor ou de chlore, ou représente un groupe phényle substitué par des atomes de chlore ou de brome, ou représente un groupe méthylbromophényle, 4-amino-3,5-dibromophényle ou ditert.-butylhydroxyphényle, et

$R_2$ représente un atome d'hydrogène, un groupe alcanoyle avec 1 à 3 atomes de carbone ou un groupe benzoyle ou phénylsulfonyle éventuellement substitué par un groupe alcoyle avec 1 à 4 atomes de carbone.

5. Sulfimines selon la revendication 1 de formule générale:

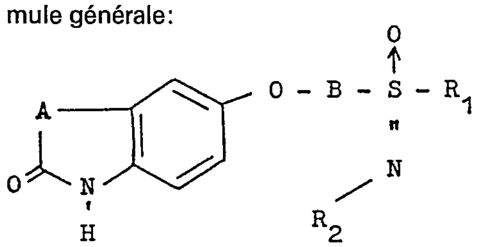 (Ia)

dans laquelle:

A représente le groupe éthylène ou vinylène,

B représente le groupe n-butylène,

$R_1$ représente le groupe méthyle, phényle, 4-fluorophényle, 4-chlorophényle, 3,4-dichlorophényle ou 3-méthyl-4-bromophényle, et

$R_2$ représente un atome d'hydrogène, le groupe acétyle ou p-toluènesulfonyle.

6. Le 6-[4-(N-acétyl-3,4-dichlorophénylsulfoximino)-butoxy]-3,4-dihydrocarbostyrile.

7. Le 6-[4-(3-méthyl-4-bromophénylsulfoximino)-butoxy]-carbostyrile.

8. Médicament contenant un composé selon les revendications 1 à 7 conjointement à un ou plusieurs excipients ou diluants inertes.

9. Utilisation d'un composé selon les revendications 1 à 7 pour la préparation d'un médicament pour la lutte contre la maladie thrombo-embolique, pour la prophylaxie de l'artériosclérose et pour la prophylaxie des métastases.

10. Procédé pour la préparation des composés selon les revendications 1 à 7, caractérisé en ce que:

a) pour la préparation d'un composé de formule générale I, dans laquelle n représente le nombre 1 et $R_2$ représente un atome d'hydrogène, on fait réagir un sulfoxyde de formule générale:

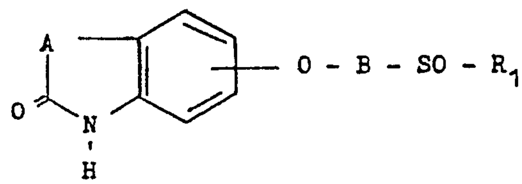 (II)

dans laquelle:

A, B, et $R_1$ sont définis comme dans les revendications 1 à 7, avec de l'acide azothydrique éventuellement formé dans le mélange réactionnel, ou

b) pour la préparation d'un composé de formule générale I, dans laquelle $R_2$ représente un atome d'hydrogène, on fait réagir un composé de formule générale:

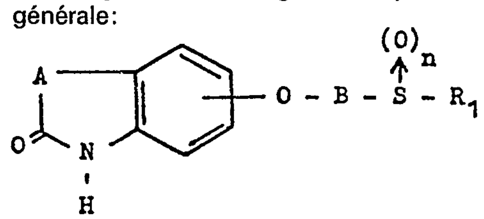 (IIa)

dans laquelle:

A, B, $R_1$ et n sont définis comme dans les revendications 1 à 7, avec un composé de formule générale:

$$H_2NO-X-R_3 \qquad (III)$$

dans laquelle:

X représente un groupe carbonyle ou sulfonyle, et

$R_3$ représente un groupe aryle disubstitué en position o tel qu'un groupe 2,4,6-triméthyl- ou triisopropylphényle ou également un groupe hydroxy, dans le cas où X représente le groupe sulfonyle, ou

c) pour la préparation d'un composé de formule générale I dans laquelle n représente le nombre 1, on oxyde un composé mercapto de formule générale:

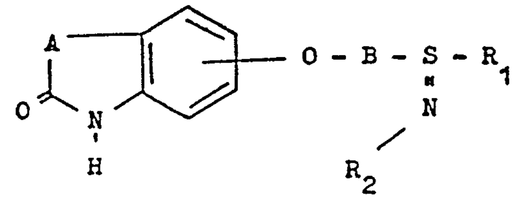 (IV)

dans laquelle:

A, B, $R_1$ et $R_2$ sont définis comme dans les revendications 1 à 7, ou

d) pour la préparation d'un composé de formule générale I, dans laquelle $R_2$ ne représente pas un atome d'hydrogène, on acyle un composé de formule générale:

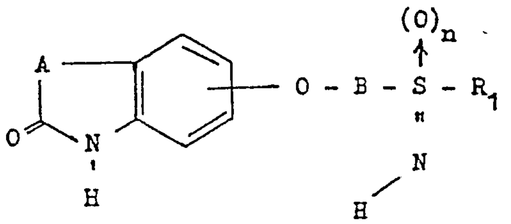 (V)

dans laquelle:

n, A, B et $R_1$ sont définis comme dans les revendications 1 à 7, ou

e) on fait réagir un composé hydroxy de formule générale:

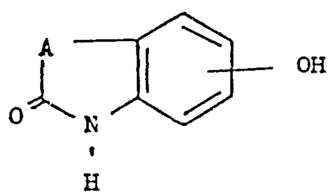 (VI)

dans laquelle:

A est défini comme dans les revendications 1 à 7, ou ses sels avec des bases minérales ou organiques tertiaires, avec un composé de formule générale:

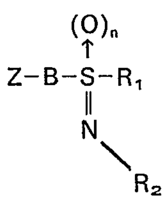 (VII)

dans laquelle:

n, $R_1$, $R_2$ et B sont définis comme dans les revendications 1 à 7, et

Z représente un groupe partant nucléophile, ou

f) pour la préparation d'un composé de formule générale I, dans laquelle n représente le nombre 0, on fait réagir un thioéther de formule générale:

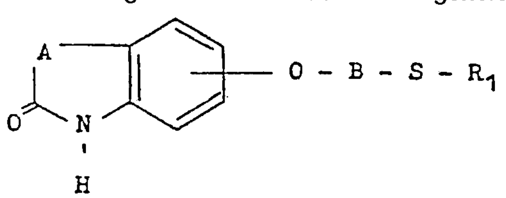 (VIII)

dans laquelle:

A, B et $R_1$ sont définis comme dans les revendications 1 à 7, avec un amide de formule générale:

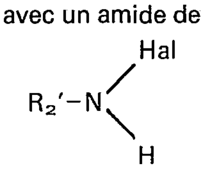 (IX)

dans laquelle:

Hal représente un atome de chlore ou de brome, et

$R_2'$ possède les significations mentionnées pour $R_2$ dans les revendications 1 à 7 à l'exception de l'hydrogène, ou avec son sel alcalin et on hydrolyse éventuellement ensuite le produit de réaction ainsi obtenu

et, si on le désire ensuite, on transforme un composé de formule générale I obtenu selon l'invention au moyen d'un acide fort en son sel ou en son sel physiologiquement supportable.

**Revendications** pour l'Etat contractant: AT

1. Procédé pour la préparation de nouvelles sulfimines de formule générale:

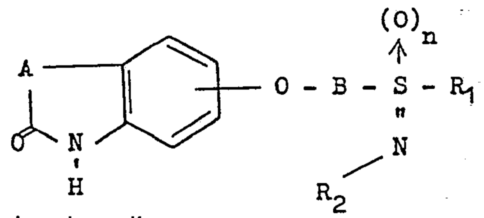 (I)

dans laquelle:

n représente le nombre 0 ou 1,

A représente un groupe méthylène, vinylène ou éthylène éventuellement substitué par un ou deux groupes alcoyle avec chacun 1 à 3 atomes de carbone,

B représente un groupe alcoylène rectiligne ou ramifié avec 2 à 6 atomes de carbone,

$R_1$ représente un groupe alcoyle avec 1 à 3 atomes de carbone éventuellement substitué par un groupe phényle, ou représente un groupe phényle, le noyau phényle pouvant dans chaque cas être substitué par un groupe alcoyle avec 1 à 4 atomes de carbone, un atome d'halogène, un groupe alcoxy avec 1 à 3 atomes de carbone, un groupe cyclohexyle, phényle ou halogénophényle, ou représente un groupe alcoyle avec 4 à 7 atomes de carbone, un groupe cycloalcoyle avec 3 à 7 atomes de carbone, un groupe phényle di- ou trisubstitué ou hydroxy- ou aminophényle disubstitué par des groupes alcoyle avec 1 à 4 atomes de carbone, des groupes alcoxy avec 1 à 3 atomes de carbone et/ou des atomes d'halogène, les substituants du noyau phényle pouvant être identiques ou différents, ou représente un groupe naphtyle éventuellement substitué par un groupe alcoxy avec 1 à 3 atomes de carbone, ou représente un groupe pyridyle, et

$R_2$ représente un atome d'hydrogène, un groupe alcanoyle avec 1 à 8 atomes de carbone éventuellement substitué par un groupe méthoxy, un groupe benzoyle ou phénylsulfonyle éventuellement substitué par un atome d'halogène, un groupe cyano ou un groupe alcoyle avec 1 à 4 atomes de carbone, ou représente un groupe alcoxy carbonyle avec en tout 2 à 4 atomes de carbone, un groupe aminocarbonyle éventuellement substitué par un groupe chlorophényle, par un ou deux groupes méthyle, ou représente un groupe naphtoyle, pinanoyle, camphosulfonyle, pentaméthyl-phénylsulfonyle, pyridinoyle ou thénoyle, et leurs sels avec des acides forts, caractérisé en ce que:

a) pour la préparation d'un composé de formule générale I, dans laquelle n représente le nombre 1 et $R_2$ représente un atome d'hydrogène, on fait réagir un sulfoxyde de formule générale:

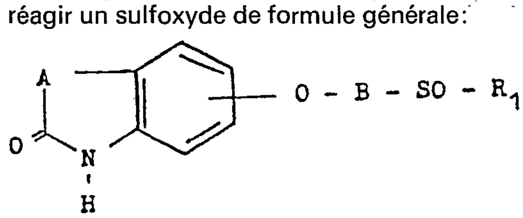 (II)

dans laquelle:

A, B et $R_1$ sont définis comme au début, avec de l'acide azothydrique éventuellement formé dans le mélange réactionnel, ou

b) pour la préparation d'un composé de formule générale I dans laquelle $R_2$ représente un atome d'hydrogène, on fait réagir un composé de formule générale:

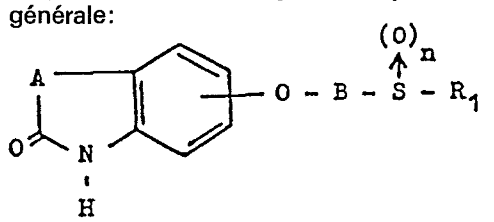
(IIa)

dans laquelle:

A, B, $R_1$ et n sont définis comme au début, avec un composé de formule générale:

$$H_2NO-X-R_3 \qquad (III)$$

dans laquelle:

X représente un groupe carbonyle ou sulfonyle, et

$R_3$ représente un groupe aryle disubstitué en position o tel qu'un groupe 2,4,6-triméthyl- ou triisopropylphényle ou également un groupe hydroxy, dans le cas où X représente le groupe sulfonyle, ou

c) pour la préparation d'un composé de formule générale I dans laquelle n représente le nombre 1, on oxyde un composé mercapto de formule générale:

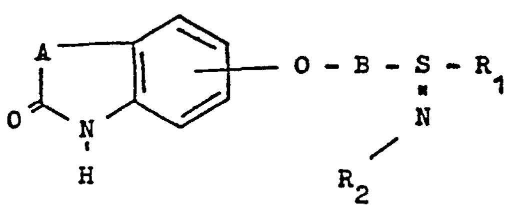
(IV)

dans laquelle:

A, B, $R_1$ et $R_2$ sont définis comme au début, ou

d) pour la préparation d'un composé de formule générale I dans laquelle $R_2$ ne représente pas un atome d'hydrogène, on acyle un composé de formule générale:

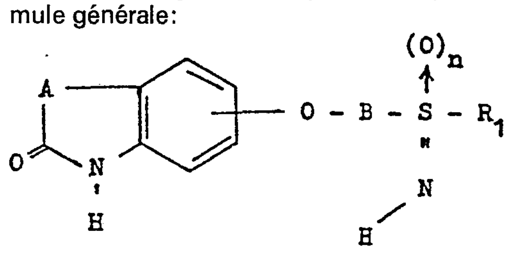
(V)

dans laquelle:

n, A, B et $R_1$ sont définis comme au début, ou

e) on fait réagir un composé hydroxy de formule générale:

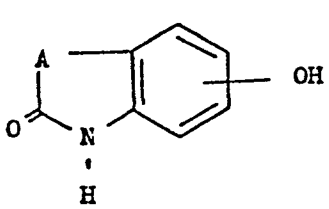
(VI)

dans laquelle:

A est défini comme au début, ou ses sels avec des bases minérales ou organiques tertiaires, avec un composé de formule générale:

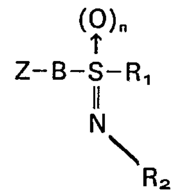
(VII)

dans laquelle:

n, $R_1$, $R_2$ et B sont définis comme au début, et

Z représente un groupe partant nucléophile, ou

f) pour la préparation d'un composé de formule générale I dans laquelle n représente le nombre 0, on fait réagir un thioéther de formule générale:

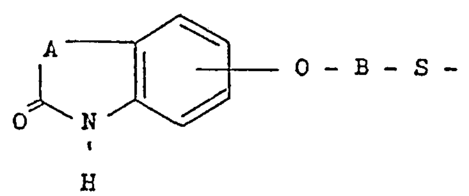
(VIII)

dans laquelle:

A, B et $R_1$ sont définis comme au début, avec un amide de formule générale:

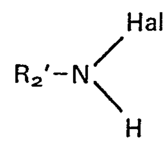
(IX)

dans laquelle:

Hal représente un atome de chlore ou de brome, et

$R_2'$ possède les significations mentionnées au début pour $R_2$ à l'exception de l'hydrogène, ou avec son sel alcalin, et, éventuellement ensuite, on hydrolyse le produit de réaction ainsi obtenu et, si on le désire ensuite, on transforme un composé de formule générale I obtenu selon l'invention au moyen d'un acide fort en son sel ou en son sel physiologiquement supportable.

2. Procédé selon la revendication 1 pour la préparation de nouvelles sulfoximines de formule générale:

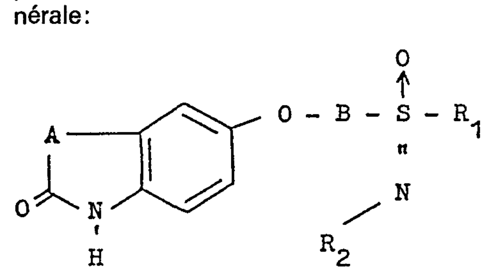
(Ia)

dans laquelle:

A, B, $R_1$ et $R_2$ sont définis comme dans la revendication 1, caractérisé en ce que:

a) pour la préparation d'un composé de formule générale Ia, dans laquelle $R_2$ représente un atome d'hydrogène, on fait réagir un sulfoxyde de formule générale:

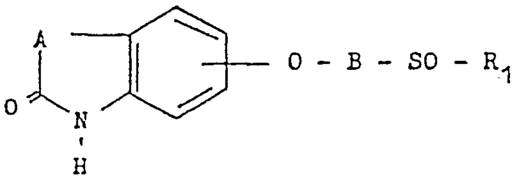 (II)

dans laquelle:

A, B, $R_1$ sont définis comme dans la revendication 1, avec de l'acide azothydrique éventuellement formé dans le mélange réactionnel, ou

b) pour la préparation d'un composé de formule générale Ia, dans laquelle $R_2$ représente un atome d'hydrogène, on fait réagir un sulfoxyde de formule générale:

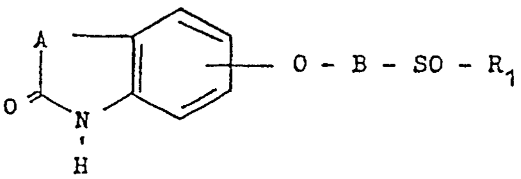 (II)

dans laquelle:

A, B et $R_1$ sont définis comme dans la revendication 1, avec un composé de formule générale:

$$H_2NO{-}X{-}R_{3a} \qquad (IIIa)$$

dans laquelle:

X est défini comme dans la revendication 1, et

$R_{3a}$ représente un groupe aryle disubstitué en position o tel qu'un groupe 2,4,6-triméthyl- ou triisopropylphényle, ou

c) on oxyde un composé mercapto de formule générale:

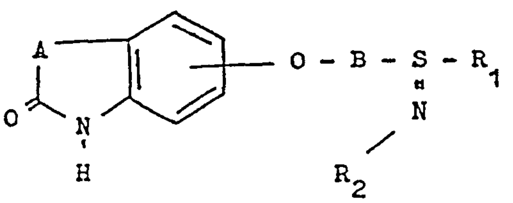 (IV)

dans laquelle:

A, B, $R_1$ et $R_2$ sont définis comme dans la revendication 1, ou

d) pour la préparation d'un composé de formule générale I, dans laquelle $R_2$ ne représente pas un atome d'hydrogène, on acyle un composé de formule générale:

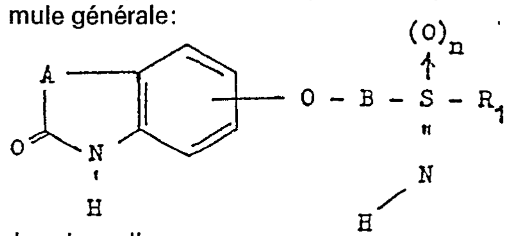 (V)

dans laquelle:

A, B et $R_1$ sont définis comme dans la revendication 1.

Priorité: 25.07.1981 (P 31 29 444.8).

3. Procédé selon les revendications 1 et 2, caractérisé en ce que la réaction est effectuée dans un solvant.

4. Procédé selon les revendications 1a, 2a et 3, caractérisé en ce que la réaction est effectuée à des températures entre 0 et 40° C.

5. Procédé selon les revendications 1b, 2b et 3, caractérisé en ce que la réaction est effectuée à des températures entre 0 et 50° C.

6. Procédé selon les revendications 1c, 2c et 3, caractérisé en ce que l'oxydation est effectuée avec un équivalent de l'agent d'oxydation utilisé et à des températures entre −80 et 100° C.

7. Procédé selon les revendications 1d, 2d et 3, caractérisé en ce que la réaction est effectuée à des températures entre −25 et 100° C.

8. Procédé selon les revendications 1e, 1f et 3, caractérisé en ce que la réaction est effectuée en présence d'une base alcaline.

9. Procédé selon les revendications 1e, 3 et 8, caractérisé en ce que la réaction est effectuée à des températures entre 0° C et la température d'ébullition du solvant utilisé.

10. Procédé selon les revendications 1f, 3 et 8, caractérisé en ce que la réaction est effectuée à des températures entre 0 et 80° C.